# EUROPEAN PATENT APPLICATION

(11) **EP 3 315 125 A1**
(43) Date of publication of application: **02.05.2018**
(21) Application number: 16196627.0
(22) Date of filing: 31.10.2016
(51) Int. Cl.: A61K 9/51, A61K 9/127, A61K 38/00, A61K 31/7088, A61K 31/713

(54) **LIPID NANOPARTICLE FORMULATION**

(71) Applicant: Silence Therapeutics (London) Ltd, London W1W 8DH (GB)
(72) Inventor: The designation of the inventor has not yet been filed
(74) Representative: CMS Cameron McKenna Nabarro Olswang LLP

(57) **Abstract**

The present invention relates to formulations comprising cationic lipids, including novel cationic lipids, in combination with other lipid components such as cholesterol, fusogenic co-lipids, and PEG-lipids to form lipid nanoparticles with nucleotides to facilitate cellular uptake.

## Description

### Field of the Invention

This invention relates to formulations comprising cationic lipids, including novel cationic lipids, in combination with other lipid components such as cholesterol, fusogenic co-lipids, and PEG-lipids to form lipid nanoparticles with nucleotides to facilitate cellular uptake.

### Background of the Invention

The delivery of active compounds into target cells remains a challenge for therapeutics. In particular, both molecular biology as well as molecular medicine heavily rely on the introduction of biologically active compounds into cells. Such biologically active compounds typically comprise, among others, DNA, RNA, as well as peptides and proteins. The barrier which has to be overcome is typically a lipid bilayer which has a negatively charged outer surface. In the art, a number of technologies have been developed to penetrate the cellular membrane and to thus introduce the biologically active compounds.

One approach for delivering biologically active compounds is the use of so-called liposomes (Bangham, J. Mol. Biol. 13, 238-252). Liposomes are vesicles which are generated upon association of amphiphilic lipids in water. Liposomes typically comprise concentrically arranged bilayers of phospholipids. Depending on the number of bilayers liposomes can be categorised as small unilamelar vesicles, multilamelar vesicles and large multilamelar vesicles. Liposomes have proven to be effective delivery agents as they allow hydrophilic compounds to be incorporated into the aqueous intermediate layers, whereas hydrophobic compounds are incorporated into the lipid layers. It is well known in the art that both the composition of the lipid formulation as well as its method of preparation have an effect on the structure and size of the resultant lipid aggregates and thus on the liposomes.

Cationic lipids are largely composed of an amine moiety and a lipid moiety, wherein the amine moiety, which shows cationicity, electrostatically interacts with a polyanion nucleic acid to form a positively-charged liposome or lipid membrane structure, thereby promoting uptake into cells and allowing for delivery of the nucleic acid.

Cationic lipids have, apart from being components of liposomes, also attracted considerable attention as they may as such be used for cellular delivery of biopolymers. Using cationic lipids, any anionic compound can be encapsulated essentially in a quantitative manner due to electrostatic interaction. In addition, it is believed that the cationic lipids interact with the negatively charged cell membranes initiating cellular membrane transport. It has been found that the use of a liposomal formulation containing cationic lipids or the use of cationic lipids as such together with a biologically active compound requires a heuristic approach as each formulation is of limited use because it typically can deliver plasmids into some but not all cell types, usually in the absence of serum.

Charge and/or mass ratios of lipids and the biologically active compounds to be transported by them have turned out to be a crucial factor in the delivery of different types of said biologically active compounds. For example, it has been shown that lipid formulations suitable for delivery of plasmids comprising 5,000 to 10,000 bases in size, are generally not effective for the delivery of oligonucleotides such as synthetic ribozymes or antisense molecules typically comprising about 10 to about 50 bases. In addition, it has recently been indicated that optimal delivery conditions for antisense oligonucleotides and ribozymes are different, even in the same cell type.

US patent 6,395,713 discloses cationic lipid based compositions which typically consist of a lipophilic group, a linker and a head group and the use of such compositions for transferring biologically active compounds into a cell.

There is, therefore, a need for improved formulations for delivering biologically active compounds to cells. In particular, there is a need for improved formulations which have the capacity to target different tissue and/or cell types to ensure that their cargo (or "payload" i.e. the biologically active compound they contain) is effectively delivered to a particular target cell for the desired therapeutic effect.

The problem underlying the present invention was to provide a means for introducing biologically active compounds into specific cell types, preferably animal liver cells. A further problem underlying the present invention is to provide a delivery agent for nucleic acids, particularly small nucleic acids such as mRNA, siRNA, siNA and RNAi (RNA interfering molecules) or aptamers and spiegelmers as well as gRNAs and components of the CRISPR-based gene editing system but not limited to RNA.

These problems are solved by the subject matter of the independent claims attached hereto. Preferred embodiments may be taken from the attached claims dependent thereon.

### Summary of the Invention

The present invention is related to formulations comprising cationic lipids, compositions containing the same and use thereof as well as a method for transferring compounds into cells.

In a first aspect of the invention there is provided a formulation comprising:
i) a cationic lipid, or a pharmaceutically acceptable salt thereof;
ii) a steroid;
iii) a phosphatidylethanolamine phospholipid;
iv) a PEGylated lipid.

In one embodiment, the cationic lipid is an amino cationic lipid.

In a further aspect of the invention there is provided an cationic lipid having the formula (I): 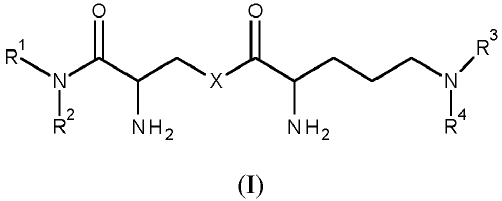 or a pharmaceutically acceptable salt thereof, wherein:
X represents O, S or NH;
R¹ and R² each independently represents a C₄-C₂₂ linear or branched alkyl chain or a C₄-C₂₂ linear or branched alkenyl chain with one or more double bonds, wherein the alkyl or alkenyl chain optionally contains an intervening ester, amide or disulfide; when X represents S or NH, R³ and R⁴ each independently represent hydrogen, methyl, ethyl, a mono- or polyamine moiety, or R³ and R⁴ together form a heterocyclyl ring; when X represents O, R³ and R⁴ each independently represent hydrogen, methyl, ethyl, a mono- or polyamine moiety, or R³ and R⁴ together form a heterocyclyl ring, or R³ represents hydrogen and R⁴ represents C(NH)(NH₂).

In one embodiment, the cationic lipid has the formula (IA): 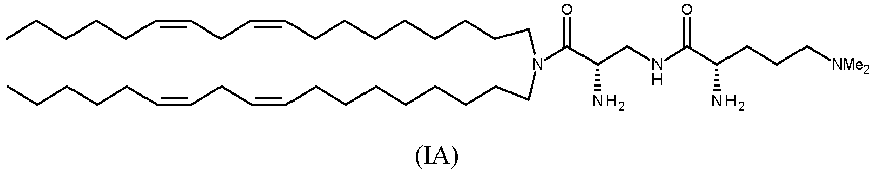 or a pharmaceutically acceptable salt thereof.

In another embodiment, the cationic lipid has the formula (IB): 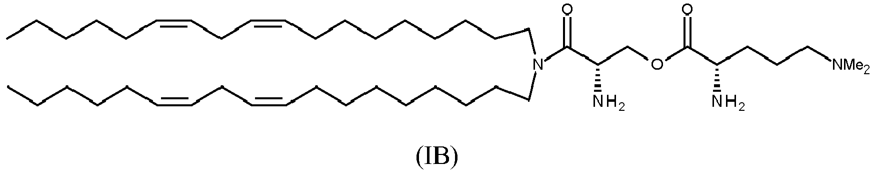 or a pharmaceutically acceptable salt thereof.

In one aspect, there is provided compounds of formulas (I), (IA) and (IB) for use in therapy. In another aspect, there is provided compounds of formulas (I), (IA) and (IB) for use in the treatment of liver disease or liver-related disease.

In the formulations of the invention, the content of the cationic lipid component may be from about 55 mol% to about 65 mol% of the overall lipid content of the formulation. In particular, the cationic lipid component is about 59 mol% of the overall lipid content of the formulation.

The formulations of the present invention further comprise a steroid. In one embodiment the steroid is cholesterol. The content of the steroid is from about 26 mol% to about 35 mol% of the overall lipid content of the lipid formulation. More particularly, the content of steroid is about 30 mol% of the overall lipid content of the lipid formulation.

The phosphatidylethanolamine phospholipid may be selected from group consisting of 1,2-diphytanoyl-*sn*-glycero-3-phosphoethanolamine (DPhyPE), 1,2-dioleoyl-*sn-*glycero-3-phosphoethanolamine (DOPE), 1,2-distearoyl-*sn*-glycero-3-phosphoethanolamine (DSPE), 1,2-Dilauroyl-sn-glycero-3-phosphoethanolamine (DLPE), 1,2-Dimyristoyl-sn-glycero-3-phosphoethanolamine (DMPE), 1,2-Dipalmitoyl-sn-glycero-3-phosphoethanolamine (DPPE), 1,2-Dilinoleoyl-sn-glycero-3-phosphoethanolamine (DLoPE), 1-Palmitoyl-2-oleoyl-sn-glycero-3-phosphoethanolamine (POPE), 1,2-Dierucoyl-sn-glycero-3-phosphoethanolamine (DEPE), 1,2-Disqualeoyl-sn-glycero-3-phosphoethanolamine (DSQPE) and 1-Stearoyl-2-linoleoyl-sn-glycero-3-phosphoethanolamine (SLPE). The content of the phospholipid is about 10 mol% of the overall lipid content of the formulation.

The PEGylated lipid may be selected from the group consisting of 1,2-dimyristoyl-sn-glycerol, methoxypolyethylene glycol (DMG-PEG) and C16-Ceramide-PEG. The content of the PEGylated lipid is about 1 to 5 mol% of the overall lipid content of the formulation.

The formulation of the present invention may have a molar ratio of the components of i):ii): iii): iv) selected from 55:34:10:1; 56:33:10:1; 57:32:10:1; 58:31:10:1; 59:30:10:1; 60:29:10:1; 61:28:10:1; 62:27:10:1; 63:26:10:1; 64:25:10:1; and 65:24:10:1.

In a further aspect, there is provided a formulation as claimed in accordance with any aspect or embodiment of the invention, and a carrier. Said formulation may comprise a further constituent.

In another aspect, there is provided a pharmaceutical composition comprising a formulation in accordance with the invention and a pharmaceutically active compound. Preferably said pharmaceutical composition further comprises a pharmaceutically acceptable carrier. In one embodiment of any aspect or embodiment of the invention, the formulation or pharmaceutical composition is a lipid nanoparticle (LNP).

In one embodiment, the formulation or pharmaceutical composition in accordance with the invention is a lipid nanoparticle (LNP) formulation wherein the mean diameter of the lipid nanoparticles is a suitable size to allow the LNP to cross the fenestrated endothelium from a small blood vessel into a liver cell such as a hepatocyte in the relevant animal. Typically the fenestrated endothelium of blood vessels supplying hepatocytes, in humans, has pores ranging from 50 to 300 nm pore diameter. Accordingly, in one embodiment, a LNP formulation in accordance with the invention has a mean diameter in the range of approximately 70 to 200 nm, or more suitably, 70 to 125 nm or 80 to 90 nm. It will be appreciated that the range may be dependent on the nature of the payload/pharmaceutically or biologically active compound. Methods for measuring the mean diameter are described herein.

In another embodiment, the zeta potential of an LNP formulation in accordance with the invention is neutral. Suitable values for zeta potential are from approximately +/-10 mV. Methods for determining zeta potential are described herein.

Suitably, a pharmaceutically active compound for use in a pharmaceutical composition in accordance with the invention can be the same as the further constituent of the formulation according to the present invention. In one embodiment, the pharmaceutically active compound may be a chemical small molecule. Preferably, a pharmaceutically active compound is a biologically active compound, more preferably any biologically active compound as disclosed herein. In one embodiment, the further constituent, pharmaceutically active compound and/or biologically active compound are preferably selected from the group comprising peptides, proteins, oligonucleotides, polynucleotides and nucleic acids. In one embodiment, the pharmaceutical composition comprises more than one active component.

A peptide as preferably used herein is any polymer consisting of at least two amino acids or amino acid residues which are covalently linked to each other, preferably through a peptide bond. More preferably, a peptide consists of two to ten amino acids. A particularly preferred embodiment of the peptide is an oligopeptide which even more preferably comprises from about 10 to about 100 amino acids. Proteins as preferably used herein are polymers consisting of a plurality of amino acids or amino acid residues which are covalently linked to each other. Preferably such proteins comprise at least 100 amino acids or amino acid residues. Peptides and proteins for use in the invention include any antibodies, or any antibody-like molecule such as an artificial antibody, an antibody fragment, an antibody fusion protein or an antibody mimetic, for example, monoclonal antibodies or any fragments or derivatives thereof.

Suitably, where the biologically active compound is a nucleic acid, it may be RNA or DNA, including components of a CRISPR-based gene editing system. Reference to RNA, includes mRNA, capped or uncapped linear RNA as well as circular RNA, siRNA, saRNA, and RNAi or RNA aptamers and spiegelmers as well as gRNAs, siNA, PNA (peptide nucleic acid), LNA (locked nucleic acid), UNA (unlocked nucleic acid), TNA (threose nucleic acid), GNA (glycol nucleic acid), or any hybrids or mixtures thereof. More preferably, the nucleic acid is a functional nucleic acid. A functional nucleic acid as preferably used herein is a nucleic acid which is not a nucleic acid coding for a peptide and/or protein, respectively. Suitable functional nucleic acids include RNAi, siRNA, siNA, saRNA, antisense nucleic acid, ribozymes, aptamers, spiegelmers and gRNA.

In one embodiment, a functional nucleic acid molecule may comprise modifications. Suitably, these modifications are within the nucleic acid sequence and may contribute to stability and/or the activity of the siRNA or RNAi molecule. Suitable modifications are described, for example in EP1389637B1 - EP1527176B1, US7452987B2, US7893245B2, and US8324370B2, Such modifications include, for example, alternating 2'O-Me, alternating 2'O-Me/fluoro etc.

In one embodiment, the formulation or pharmaceutical composition in accordance with the invention comprises an siRNA as the pharmaceutically active compound, wherein the total lipid to siRNA weight ratio in the formulation or composition is in the range of 10-30:1, preferably 20:1.

The pharmaceutical composition in accordance with the invention may include an mRNA. Such an mRNA may comprises modifications such as modifications which contribute to stability of activity of the mRNA. Suitable modifications will be known to those skilled in the art. Suitably, where the formulation or composition in accordance with the invention comprises mRNA, the total lipid to mRNA weight ratio is 20-50:1, preferably 40-50:1, more preferably 45:1 (mass/mass Lipid:mRNA ratio).

In one embodiment, the pharmaceutical composition in accordance with the invention may comprise at least one nucleic acid molecule. In one embodiment, the pharmaceutical composition may comprise a combination of different nucleic acid molecules. A composition comprising different nucleic acid molecules may comprise molecules with different biological activities such as, for example, targeting different genes. In another embodiment, different biological activities may make up different components of a biological system.

Accordingly, in one embodiment, the pharmaceutical composition may comprise a CRISPR RNA (crRNA) plus a tracer RNA (tracrRNA), a guide RNA (gRNA) or a single guide RNA (sgRNA) and/or a donor DNA in conjunction with a CRISPR endonuclease. Suitably the CRISPR endonuclease may be provided as a protein or polypeptide or as an mRNA encoding said CRISPR endonuclease. A composition or formulation comprising this combination is suitable for delivering a CRISPR gene editing activity to a target cell. In one embodiment, the pharmaceutical composition or formulation in accordance with the invention may provide the gRNA and mRNA encoding a CRISPR endonuclease, for separate, sequential or simultaneous administration. That is, the gRNA and mRNA may be provided within the same formulation or lipid nanoparticle in accordance with the invention or may be provided in separate lipid nanoparticles for separate, simultaneous or sequential administration. Suitably the ratio of gRNA to mRNA for administration is 1:1, 1:3, 1:9, 1:19, for example (i.e. 50%, 25%, 10% and 5% of guide RNA).

In one embodiment, a gRNA and an mRNA encoding a CRISPR endonuclease such as cas9 are co-loaded into a formulation in accordance with the invention.

Advantageously, co-loading enables a better encapsulation efficiency to be obtained. Suitably, a formulation or pharmaceutical composition in accordance with the invention into which gRNA and mRNA are co-loaded comprises LNPs with a mean diameter of between 80 and 160 nm.

In one embodiment, the gRNA may be a modified gRNA sequence. Suitable modifications are described, for example in WO2016/089433 and PCT/GB2016/053312. Other suitable modifications will be familiar to those skilled in the art.

By "CRISPR endonuclease" is meant an endonuclease that can be used in a CRISPR gene editing composition. Suitable "CRISPR endonucleases" include cas9 and its mutants and modified forms. Accordingly, the mRNA for use in combination with a gRNA is one which encodes a CRISPR endonuclease, preferably cas9. Other "CRISPR endonucleases" include cpf1, for example. The skilled person will be aware that a gRNA pairs with a particular "CRISPR endonuclease". Accordingly, the invention contemplates a composition using a suitable gRNA/endonuclease pairing.

Suitably, a gRNA is specific for a target gene, preferably wherein the target gene is a gene associated with liver disease.

In one embodiment, the formulation or pharmaceutical composition in accordance with the invention preferentially targets cells in the liver but not in other organs (e.g. lung, kidney, heart). Liver cells include hepatocytes and hepatocyte precursors, stellate cells/pericytes, endothelial cells, Kupffer cells, macrophages and neutrophils, for example. In one embodiment, where the formulation or pharmaceutical composition comprises a gRNA in combination with an mRNA encoding a CRISPR endonuclease such as cas9, the composition preferentially targets hepatocytes, pericentral hepatocytes (which act as stem cells in healthy livers) and, or, suitably hepatocyte stem cells. The preferential targeting of cells in the liver is due to the size and neutral charge of the lipid nanoparticles. In certain instances, targeting of the liver cells may have a secondary effect on, or influence other organs in the body. It will therefore be appreciated that the formulations or pharmaceutical compositions of the present invention also have utility in the treatment of diseases other than those associated with the liver.

In another aspect, the invention provides a method for preparing a formulation or pharmaceutical composition in accordance with the invention comprising:
a) preparing a lipid stock solution comprising
   i) a cationic lipid, or a pharmaceutically acceptable salt thereof;
   ii) a steroid;
   iii) a phosphatidylethanolamine phospholipid;
   iv) a PEGylated lipid; and
b) mixing said lipid stock solution with a pharmaceutically active compound in a microfluidic mixer to obtain said formulation or pharmaceutical composition.

Suitably, the microfluidic mixing conditions are chosen so as to obtain encapsulation of the pharmaceutically active compound at an encapsulation efficiency of above 80%, preferably above 90%, more preferably above 94%.

In one aspect of the invention there is provided a pharmaceutical composition in accordance with any embodiment of the invention for use as a medicament. Accordingly, said pharmaceutical composition is suitable for use in animals, particularly in humans and other vertebrates. Suitably said pharmaceutical composition is for use, but not limited to in the treatment of liver disease or diseases where protein expression in the liver has an impact on vertebrate pathologies. As mentioned above, the pharmaceutical compositions described herein may also find use in the treatment of diseases not associated with the liver.

Liver disease or liver-related diseases in animals, more particularly humans, may include but would not be limited to congenital diseases or acquired diseases for example viral and parasite infectious diseases, oncologic pathologies such as primary tumours and metastases, metabolic and/or endocrine disorders as well as inflammatory and immune and auto-immune conditions. Liver disease may include Hepatitis C, Hepatitis B, Hepatitis, Hepatitis A, Cirrhosis, Liver Cancer, Hepatocellular Carcinoma, Hepatic Encephalopathy, Autoimmune Hepatitis, Wilson Disease, Alpha-1 Antitrypsin Deficiency, Hepatitis D, Alagille Syndrome, Portal Hypertension, Steatohepatitis, Chronic Hepatitis and Hepatitis E, for example.

Suitably any transcript, transcript family or series of different transcripts or genomic chromosomal or mitochondrial sequences including but not limited to exons and introns of genes and regulatory elements involved in any liver disease or liver-related disorder may be targeted using a composition or formulation in accordance with the invention. Such a any transcript, transcript family or series of different transcripts may be targeted by any biologically active compound as described herein. In one embodiment, the biologically active compound is a nucleic acid molecule which recognises a pathology-related transcript e.g. an mRNA, gRNA, siRNA, saRNA etc. as described herein.

Suitably any gene involved in any liver disease may be targeted using a composition or formulation in accordance with the invention. Such a gene may be targeted by any biologically active compound as described herein. In one embodiment, the biologically active compound is a nucleic acid molecule which recognises a liver disease gene e.g. an mRNA, gRNA, siRNA etc. as described herein.

### Brief Description of the Figures

**Figure 1** shows a schematic of the components of Lipid Nanoparticle Formulation 2.
**Figure 2** shows comparison of gene silencing activity in the liver following intravenous administration of exemplary Lipid Nanoparticle Formulation 1 and Lipid Nanoparticle Formulation 2.
**Figure 3** shows endothelial Tie-2 mRNA expression in the liver, lung, spleen, kidney and heart following intravenous administration of Lipid Nanoparticle Formulation 2.
**Figure 4** shows stellate cell marker GFAP mRNA expression in the liver, lung, spleen, kidney and heart following intravenous administration of Lipid Nanoparticle Formulation 2.
**Figure 5** shows hepatocyte ApoB gene silencing in mouse liver following intravenous administration of Lipid Nanoparticle Formulation 2.
**Figure 6** shows stellate-targeted gene-silencing activity of Lipid Nanoparticle Formulation 2 containing non-fluoro or flouro-modified Col1A1 siRNA in mouse liver.
**Figure 7** shows macrophage-targeted gene-silencing activity of Lipid Nanoparticle Formulation 2 in mouse liver.
**Figure 8** shows pericyte/cholangiocyte-targeted gene-silencing activity of Lipid Nanoparticle Formulation 2 in mouse liver.
**Figure 9** shows in vivo hepatocyte-TTR gene-silencing activity of Lipid Nanoparticle Formulation 2 in mouse liver.
**Figure 10** shows stem cell Glul (glutamine synthetase) positive gene-silencing activity of Lipid Nanoparticle Formulation 2 in mouse liver.
**Figure 11** shows payload-related immune response. In vivo mouse immune response to LNF2 delivering either siRNA or mRNA was quantified by measuring the cytokines TNF-alpha, IFN-gamma and IP-10.
**Figure 12** shows in vitro disruption of TTR gene in Hepa 1-6 and primary Hepatocytes after transfection with different mRNA Cas9 and sgRNA ratios.
**Figure 13** shows levels of serum PCSK9 protein in individual animals. Blood samples were taken on days 21, 35, 63, 91, 119, 147 and 175 post-injection (dpi). Serum was obtained, aliquoted and stored at -80°C. PCSK9 levels were measured by ELISA kit (days 21-175).
**Figure 14** shows levels of serum PCSK9 protein shown as (A) mean values per group for the indicated time points or (B) normalized to the sgRNA control group. Blood samples were taken on days 21, 35, 63, 91, 119, 147 and 175 post-injection (dpi). Serum was obtained, aliquoted and stored at -80°C. PCSK9 levels were measured by ELISA kit (days 21-175).
**Figure 15** shows levels of serum TTR protein in individual animals. Blood samples were taken on days 21, 35, 63, 91, 119, 147 and 175 post injection (dpi). Serum was obtained, aliquoted and stored at -80°C. TTR levels were measured by ELISA kit (days 21-175)
**Figure 16** shows levels of serum TTR protein shown as (A) mean values per group for the indicated time points or (B) normalized to the sgRNA control group. Blood samples were taken on days 21, 35, 63, 91, 119, 147 and 175 post-injection (dpi). Serum was obtained, aliquoted and stored at -80°C. TTR levels were measured by ELISA kit (days 21-175).

### Detailed Description of Invention

One aspect of the invention provides a formulation comprising:
i) a cationic lipid, or a pharmaceutically acceptable salt thereof;
ii) a steroid;
iii) a phosphatidylethanolamine phospholipid;
iv) a PEGylated lipid.

In certain embodiments disclosed herein, the ionisable lipids generally comprise a polar, hydrophilic head group and a non-polar, hydrophobic tail group. For example, the ionisable lipid compounds described herein may generally comprise one or more cationic and/or ionisable functional head groups, such as an amine functional group. In one embodiment, the ionisable lipid is an amino ionisable lipid.

Suitable cationic lipids are described in EP2781507 which is incorporated herein by reference in its entirety. In one embodiment, the cationic lipid has the structure: 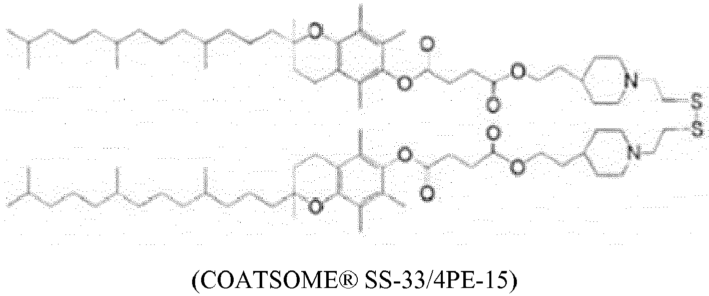 or a pharmaceutically acceptable salt thereof.

Other suitable cationic lipids are described in WO2015/199952, which is incorporated herein by reference in its entirety.

In one aspect of the invention there is provided a cationic lipid having the formula (I): 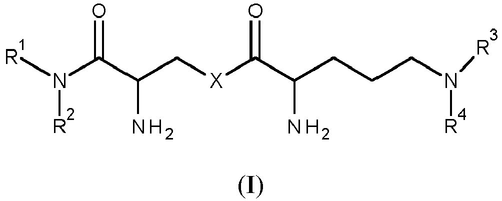 or a pharmaceutically acceptable salt thereof, wherein:
X represents O, S or NH;
R¹ and R² each independently represents a C₄-C₂₂ linear or branched alkyl or a C₄-C₂₂ linear or branched alkenyl chain with one or more double bonds, wherein the alkyl or alkenyl chain optionally contains an intervening ester, amide or disulfide;
when X represents S or NH, R³ and R⁴ each independently represent hydrogen, methyl, ethyl, mono- or polyamide, or R³ and R⁴ together form a heterocyclyl ring;
when X represents O, R³ and R⁴ each independently represent hydrogen, methyl, ethyl, mono- or polyamide, or R³ and R⁴ together form a heterocyclyl ring, or R³ represents hydrogen and R⁴ represents C(NH)(NH₂).

In one embodiment, X represents O.

In another embodiment, X represents NH.

In another embodiment, X represents S.

R¹ and R² may be the same or different. Preferably, R¹ and R² are both the same. In one embodiment, R¹ and R² each independently represent a C₄-C₂₂ alkyl chain. In another embodiment, R¹ and R² each independently represent a C₁₀-C₂₀ alkyl chain. In another embodiment, R¹ and R² each independently represent a C₁₄-C₁₈ alkyl chain. In one embodiment, R¹ and R² both represent a C₁₄ alkyl chain. In another embodiment, R¹ and R² both represent a C₁₆ alkyl chain. In yet another embodiment, R¹ and R² both represent a C₁₈ alkyl chain. The alkyl chain may be linear or branched.

In another embodiment, R¹ and R² each independently represent a C₄-C₂₂ alkenyl chain. In another embodiment, R¹ and R² each independently represent a C₁₀-C₂₀ alkenyl chain. In another embodiment, R¹ and R² each independently represent a C₁₄-C₁₈ alkenyl chain. In one embodiment, R¹ and R² both represent a C₁₄ alkenyl chain. In another embodiment, R¹ and R² both represent a C₁₆ alkenyl chain. In yet another embodiment, R¹ and R² both represent a C₁₈ alkenyl chain. The alkyl chain may be linear or branched. The alkenyl chain may have one or double bonds, for example, 1, 2, 3, 4, 5, 6, 7, 8, 9, 10 or more double bonds. In one embodiment, the alkenyl chain has one, two, three or four double bonds within the chain. In one embodiment, the alkenyl chain has one double bond within the chain. In another embodiment, the alkenyl chain has two double bonds within the chain. In another embodiment, the alkenyl chain has three double bonds within the chain. In yet another embodiment, the alkenyl chain has four double bonds within the chain. In particular, R¹ and R² may have the same number of double bonds within the alkenyl chain.

In certain embodiments, the alkyl or alkenyl chain contains one or more intervening ester, amide and/or a disulfide bond. In one embodiment, the alkyl or alkenyl chain contains an ester, i.e. a -C(O)O- or -OC(O)- moiety. Additionally or alternatively, the alkyl or alkenyl chain contains an amide, i.e. an intervening -N(H)C(O)- or-C(O)N(H)- moiety. Additionally or alternatively, the alkyl or alkenyl chain contains a disulfide, i.e. a -S-S- group.

In certain embodiments, R¹ and R² both represent C₁₈ alkenyl. In another embodiment, R¹ and R² both represent octadeca-9,12-diene.

R³ and R⁴ may be the same or different. Preferably, R³ and R⁴ are the same.

In one embodiment, both R³ and R⁴ represent hydrogen.

In another embodiment, R³ and R⁴ each independently represent methyl or ethyl. In one embodiment, both R³ and R⁴ represent methyl. In another embodiment, both R³ and R⁴ represent ethyl. In another embodiment, R³ and R⁴ each independently represent a mono- or polyamine. The term "monoamine" is defined as a group which has one amine nitrogen atom and 2 to 40 carbon atoms. The term "polyamine" is defined as group which has two or more amine nitrogen atoms and 2 to 40 carbon atoms. Examples of such groups include utrescine, cadaverine, spermidine and spermine.

Alternatively, R³ and R⁴ together form a heterocyclyl ring. In particular, the heterocyclyl ring is selected from an azetidine, pyrrolidine or piperidine ring. In another embodiment, when X is O, R³ represents hydrogen and R⁴ represents C(NH)(NH₂) i.e. a guanidine moiety.

In one embodiment, there is provided the cationic lipid of formula (I) or a pharmaceutically acceptable salt thereof, wherein:
X represents O, S or NH;
R¹ and R² each independently represent a C₁₆-C₂₀ linear or branched alkenyl chain, wherein the alkenyl chain contains 1 to 4 double bonds; and
R³ and R⁴ each represent methyl.

In one embodiment, the cationic has the formula (IA): 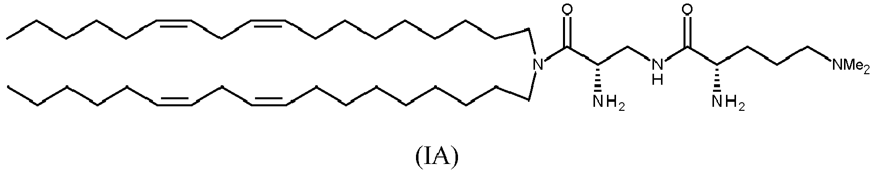 or a pharmaceutically acceptable salt thereof.

In another embodiment, the cationic lipid has the formula (IB): 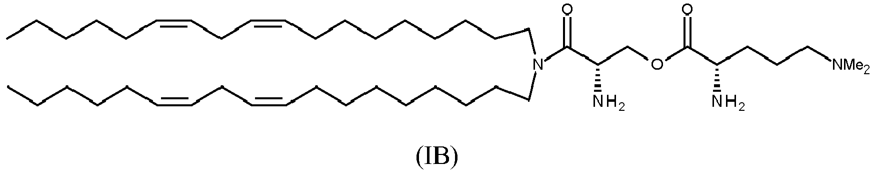 or a pharmaceutically acceptable salt thereof.

Included herein is the cationic lipid according to formula (II): 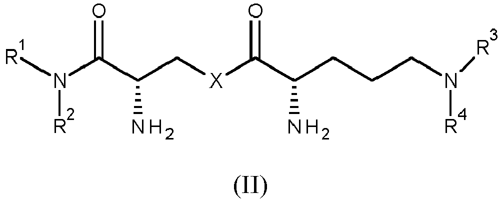 or a pharmaceutically acceptable salt thereof, wherein:
X represents O, S or NH;
R¹ and R² each independently represents a C₄-C₂₂ linear or branched alkyl chain or a C₄-C₂₂ linear or branched alkenyl chain with one or more double bonds, wherein the alkyl or alkenyl chain optionally contains an intervening ester, amide or disulfide; when X represents S or NH, R³ and R⁴ each independently represent hydrogen, methyl, ethyl, mono- or polyamine, or R³ and R⁴ together form a heterocyclyl ring; and
when X represents O, R³ and R⁴ each independently represent hydrogen, methyl, ethyl, mono- or polyamine, or R³ and R⁴ together form a heterocyclyl ring, or R³ represents hydrogen and R⁴ represents C(NH)(NH₂).

The content of the cationic lipid component may be from about 55 mol% to about 65 mol% of the overall lipid content of the formulation. For example, the cationic lipid component may be from about 56 mol% to about 64 mol%, from about 57 mol% to about 63 mol%, from about 57 mol% to about 62 mol%, from about 58 mol% to about 61 mol% of the overall lipid content of the formulation. The cationic lipid component may therefore be about 55 mol%, about 56 mol%, about 57 mol%, about 58 mol%, about 59 mol%, about 60 mol%, about 61 mol%, about 62 mol%, about 63 mol%, about 64 mol%, about 65 mol%, of the overall lipid content of the formulation.

In particular, the cationic lipid component is about 59 mol% of the overall lipid content of the formulation.

The formulations of the present invention further comprise a steroid. In one embodiment the steroid is cholesterol.

The content of the steroid is from about 26 mol% to about 35 mol% of the overall lipid content of the lipid formulation. More particularly, the content of steroid is about 30 mol% of the overall lipid content of the lipid formulation.

The phosphatidylethanolamine phospholipid may be selected from group consisting of 1,2-diphytanoyl-*sn*-glycero-3-phosphoethanolamine (DPhyPE), 1,2-dioleoyl-*sn-*glycero-3-phosphoethanolamine (DOPE), 1,2-distearoyl-*sn*-glycero-3-phosphoethanolamine (DSPE), 1,2-Dilauroyl-sn-glycero-3-phosphoethanolamine (DLPE), 1,2-Dimyristoyl-sn-glycero-3-phosphoethanolamine (DMPE), 1,2-Dipalmitoyl-sn-glycero-3-phosphoethanolamine (DPPE), 1,2-Dilinoleoyl-sn-glycero-3-phosphoethanolamine (DLoPE), 1-Palmitoyl-2-oleoyl-sn-glycero-3-phosphoethanolamine (POPE), 1,2-Dierucoyl-sn-glycero-3-phosphoethanolamine (DEPE), 1,2-Disqualeoyl-sn-glycero-3-phosphoethanolamine (DSQPE) and 1-Stearoyl-2-linoleoyl-sn-glycero-3-phosphoethanolamine (SLPE).

In one embodiment, the phospholipid 1,2-dioleoyl-*sn*-glycero-3-phosphoethanolamine. In another embodiment, the phospholipid is 1,2-distearoyl-*sn*-glycero-3-phosphoethanolamine. In yet another embodiment, the phospholipid is 1,2-distearoyl-*sn*-glycero-3-phosphoethanolamine. In yet another embodiment, the phospholipid is 1,2-Dilauroyl-sn-glycero-3-phosphoethanolamine. In a further embodiment, the phospholipid is 1,2-Dimyristoyl-sn-glycero-3-phosphoethanolamine. In yet a further embodiment, the phospholipid is 1,2-Dipalmitoyl-sn-glycero-3-phosphoethanolamine. In an even further embodiment, the phospholipid is 1,2-Dilinoleoyl-sn-glycero-3-phosphoethanolamine. In yet another embodiment, the phospholipid is 1-Palmitoyl-2-oleoyl-sn-glycero-3-phosphoethanolamine. In another embodiment, the phospholipid is 1,2-Dierucoyl-sn-glycero-3-phosphoethanolamine. In yet another embodiment, the phospholipid is 1,2-Disqualeoyl-sn-glycero-3-phosphoethanolamine. In yet another embodiment, the phospholipid is 1-Stearoyl-2-linoleoyl-sn-glycero-3-phosphoethano lamine.

The content of the phospholipid is about 10 mol% of the overall lipid content of the formulation.

Suitable PEGylated lipids include, but are not limited to 1,2-dimyristoyl-sn-glycerol, methoxypolyethylene glycol (DMG-PEG), N-palmitoyl-sphingosine-1-{succinyl[methoxy(polyethylene glycol) (C16-Ceramide-PEG), 1,2-dipalmitoyl-sn-glycero-3-phosphoethanolamine-N-methoxy(polyethylene glycol) (DPPE-PEG), 1,2-dimyristoyl-sn-glycero-3-phosphoethanolamine-N-methoxy(polyethylene glycol) (DMPE-PEG), 1,2-distearoyl-sn-glycero-3-phosphoethanolamine-N-[methoxy(polyethylene glycol)-2000] (DSPE-PEG), 2-dimyristoyl-sn-glycerol, methoxypolyethylene glycol (DMG-PEG1) and 1,2-Distearoyl-sn-glycerol, methoxypolyethylene Glycol (DSG-PEG).

Other suitable PEGylated lipids are disclosed in US9006417 and WO2015/199952, which are incorporated herein by reference.

In particular, the PEGylated lipid may be selected from the group consisting of 1,2-dimyristoyl-sn-glycerol, methoxypolyethylene glycol (DMG-PEG) and N-palmitoyl-sphingosine-1-{succinyl[methoxy(polyethylene glycol) (C16-Ceramide-PEG).

The content of the PEGylated lipid is from about 1 mol% to about 5 mol% of the overall lipid content of the formulation. In particular, the content of the PEGylated lipid is from about 1% to about 3 mol% of the overall lipid content of the formulation. More particularly, the content of the PEGylated lipid is about 1 mol% of the overall lipid content of the formulation.

The formulation of the present invention may have a molar ratio of the components of i):ii): iii): iv) selected from 55:34:10:1; 56:33:10:1; 57:32:10:1; 58:31:10:1; 59:30:10:1; 60:29:10:1; 61:28:10:1; 62:27:10:1; 63:26:10:1; 64:25:10:1; and 65:24:10:1.

A reference to a cationic lipid, including a compound of formula (I) and sub-groups thereof (i.e. formulas (IA), (IB) and (II)), also includes ionic forms, salts, solvates, isomers (including geometric and stereochemical isomers (racemates, racemic mixtures and individual stereoisomers)), tautomers, N-oxides, esters and isotopes. The atoms in the cationic lipids described herein, including the compounds of formula (I) and sub-groups thereof, may exhibit their natural isotopic abundance, or one or more of the atoms may be artificially enriched in a particular isotope having the same atomic number, but an atomic mass or mass number different from the atomic mass or mass number predominantly found in nature, and includes all suitable isotopic variations of the cationic lipids described herein, including compounds of formula (I) and sub-groups thereof. For example, different isotopic forms of hydrogen include ¹H, ²H (D), and ³H (T). Protium (¹H) is the predominant hydrogen isotope found in nature. Enriching for deuterium (²H) may afford certain therapeutic advantages such as increasing *in vivo* half-life or reducing dosage requirements.

The cationic lipids described herein can exist in the form of salts, for example acid addition salts or, in certain cases salts of organic and inorganic bases such as carboxylate, sulfonate and phosphate salts. All such salts are within the scope of this invention, and references to cationic lipids and compounds of formula (I) and sub-groups thereof include the salt forms of the compounds.

Pharmaceutical acceptable salts of the basic cationic lipid derived from inorganic or organic acids. For example from inorganic acids such as hydrochloric, hyrdobromic, sulfuric, sulfamic, phosphoric, nitric acid and the like, as well as, salts from organic acids such as acetic, propanoic, succinic, glcolic, stearic , lactic, malic, tartaric, citric, ascorbic, pamoic, maleic, hdroxymaleic, phenylacetic, glutamic, benzoic, salicylic, sulfanilic, 2-acetoxybenzoic, fumatic, tolenesulfonic, methanesulfonic, ethanesulfonic, naphthalenesulfonic, benzenesulfonic, trifluoroacetic and the like.

In a further aspect, there is provided a formulation as claimed in accordance with any aspect or embodiment of the invention, and a carrier. Said formulation may comprise a further constituent.

In another aspect, there is provided a pharmaceutical composition comprising a formulation in accordance with the invention and a pharmaceutically active compound. Preferably said pharmaceutical composition further comprises a pharmaceutically acceptable carrier. In one embodiment of any aspect or embodiment of the invention, the formulation or pharmaceutical composition is a lipid nanoparticle (LNP).

The pharmaceutically active compound and/or further constituent may be selected from the group comprising peptides, proteins, oligonucleotides, polynucleotides and nucleic acids.

Examples of suitable carriers, are known to those skilled in the art and include but are not limited to preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms. The compositions may be in the form of, for example, tablets, capsules, powders, granules, elixirs, lozenges, suppositories, syrups and liquid preparations including suspensions and solutions. The term "pharmaceutical composition" in the context of this invention means a composition comprising an active agent and comprising additionally one or more pharmaceutically acceptable carriers. The composition may further contain ingredients selected from, for example, diluents, adjuvants, excipients, vehicles, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms.

siRNA are small interfering RNA as, for example, described in international patent application PCT/EP03/08666. These molecules typically consist of a double-stranded RNA structure which comprises between 15 and 25, preferably 18 to 23 nucleotide pairs which are capable of base-pairing to each other, i. e. are essentially complementary to each other, typically mediated by Watson-Crick base-pairing. One strand of this double-stranded RNA molecule is essentially complementary to a target nucleic acid, preferably an mRNA, whereas the second strand of said double-stranded RNA molecule is essentially identical to a stretch of said target nucleic acid. The siRNA molecule may be flanked on each side and each stretch, respectively, by a number of additional nucleotides which, however, do not necessarily have to base-pair to each other.

RNAi has essentially the same design as siRNA, however, the molecules are significantly longer compared to siRNA. RNAi molecules typically comprise 50 or more nucleotides and base pairs, respectively.

A further class of functional nucleic acids which are active based on the same mode of action as siRNA and RNAi is siNA. siNA is, e.g., described in international patent application PCT/EP03/074654. More particularly, siNA corresponds to siRNA, whereby the siNA molecule does not comprise any ribonucleotides.

Antisense nucleic acids, as preferably used herein, are oligonucleotides which hybridise based on base complementarity with a target RNA, preferably mRNA, thereby activating RNaseH. RNaseH is activated by both phosphodiester and phosphothioate-coupled DNA. Phosphodiester-coupled DNA, however, is rapidly degraded by cellular nucleases although phosphothioate-coupled DNA is not. Antisense polynucleotides are thus effective only as DNA-RNA hybrid complexes. Preferred lengths of antisense nucleic acids range from 16 to 23 nucleotides. Examples for this kind of antisense oligonucleotides are described, among others, in US patent 5,849,902 and US patent 5,989,912.

A further group of functional nucleic acids are ribozymes which are catalytically active nucleic acids preferably consisting of RNA which basically comprises two moieties. The first moiety shows a catalytic activity, whereas the second moiety is responsible for the specific interaction with the target nucleic acid. Upon interaction between the target nucleic acid and the said moiety of the ribozyme, typically by hybridisation and Watson-Crick base-pairing of essentially complementary stretches of bases on the two hybridising strands, the catalytically active moiety may become active which means that it cleaves, either intramolecularly or intermolecularly, the target nucleic acid in case the catalytic activity of the ribozyme is a phosphodiesterase activity. Ribozymes, the use and design principles are known to the ones skilled in the art and, for example, described in Doherty and Doudna (Annu. Ref. Biophys. Biomolstruct. 2000; 30: 457-75).

A still further group of functional nucleic acids are aptamers. Aptamers are D-nucleic acids which are either single-stranded or double-stranded and which specifically interact with a target molecule. The manufacture or selection of aptamers is, e.g., described in European patent EP 0 533 838. In contrast to RNAi, siRNA, siNA, antisense-nucleotides and ribozymes, aptamers do not degrade any target mRNA but interact specifically with the secondary and tertiary structure of a target compound such as a protein. Upon interaction with the target, the target typically shows a change in its biological activity. The length of aptamers typically ranges from as little as 15 to as much as 80 nucleotides, and preferably ranges from about 20 to about 50 nucleotides.

Another group of functional nucleic acids are spiegelmers as, for example, described in international patent application WO 98/08856. Spiegelmers are molecules similar to aptamers. However, spiegelmers consist either completely or mostly of L-nucleotides rather than D-nucleotides in contrast to aptamers. Otherwise, particularly with regard to possible lengths of spiegelmers, the same applies to spiegelmers as outlined in connection with aptamers.

In one aspect, the compounds, formulations and compositions disclosed herein are for use in therapy. In another aspect, the compounds, formulations and compositions of the invention are for use in the treatment of liver disease or liver-related disorders.

The compounds, formulations and compositions described herein may be used in the manufacture of a medicament for the treatment of liver disease or liver-related disorders.

In a further aspect there is provided a method of treatment or prevention of liver disease or liver-related disorders, the method comprising administering a pharmaceutically effective amount of a compound, formulation or composition described herein to an individual in need thereof.

Various further aspects and embodiments of the present invention will be apparent to those skilled in the art in view of the present disclosure.

All documents mentioned in this specification are incorporated herein by reference in their entirety.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures described above and tables described below.

### Examples

### a) Preparation of Lipid Nanoparticle formulations

### i) Lipid Nanoparticle Formulation 1

### In some examples Lipid Nanoparticle Formulation 1 composition was employed:

Cationic lipid (Coatsome SS-33/4PE-15, TS-P4C2 described in US2014/00035157), cholesterol, 1,2-distearoyl-*sn*-glycero-3-phosphocholine (DSPC, from Avanti) and DMG-PEG (2kDa, from NOF Corporation) were solubilized in alcoholic solution at a molar ratio of 50:38.5:10:1.5. Coatsome SS-33/4PE-15 was prepared at 30 mg/ml in t-ButOH, all others at 20 mg/ml, dissolved in ethanol and filter sterilized through 0.2 µm syringe filters.

### ii) Lipid Nanoparticle Formulation 2

### In other examples, Lipid Nanoparticle Formulation 2 composition was employed:

Cationic lipid (Coatsome SS-33/4PE-15), cholesterol, DPhyPE, and PEG-lipid were solubilized in alcoholic solution at a molar ratio of 59:30:10:1. Coatsome SS-33/4PE-15 was prepared at 30 mg/ml in t-ButOH, all others at 20 mg/ml, dissolved in ethanol and filter sterilized using 0.2 µm syringe filters.

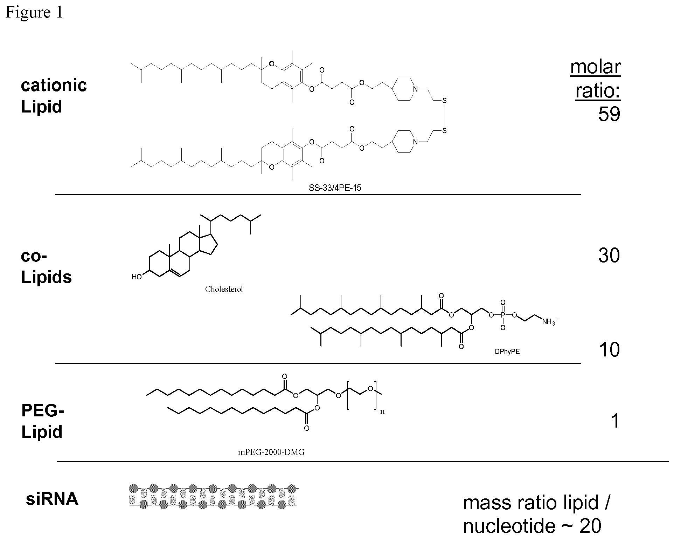

Structures of the components of the Lipid Nanoparticle Formulations are shown in Figure 1.

### b) siRNA formulations - LNP preparation

LNP were prepared by mixing appropriate volumes of lipid stock solutions in ethanol buffer with an aqueous phase containing appropriate amounts of siRNA employing a microfluidic mixer as NanoAssemblr (Precision Nanosystems).

For the encapsulation of siRNA, the desired amount of such mixtures was dissolved in 25 mM sodium acetate, pH 4.0. Then, 3 volumes of the lipid in ethanol and 1 volume of the nucleic acids in water were combined in the microfluidic mixer (NanoAssemblr, Precision Nanosystems, Canada). The lipid mixture was then dialyzed for 4 hours against 10 mM HEPES 9% Sucrose (3 cycles) using dialysis membranes (molecular weight cutoff 3500 Da, Slide-A-Lyzer™ Dialysis Cassettes, Thermofisher). The mean diameter and zeta potential of the LNP after dialysis was as determined by dynamic light scattering and Laser Doppler Micro-electrophoresis, respectively using a Zetasizer Nano ZS (Malvern). Encapsulation efficiency (EE%) was calculated by the following equation % encapsulation = (Ft - Fi)/Ft x 100. Whereby Fi = free unencapsulated RNA as determined by addition of RiboGreen (Molecular Probes, Eugene, OR, USA) to the LNP aliquot , and Ft = total content RNA content measured by adding RiboGreen (Molecular Probes, Eugene, OR, USA) to fluorescence value = Fi) to an aliquot of lysed LNP achieved by incubation with 1% Triton X-100.

The siRNA was modified with a proprietary pattern for enhanced stability as described, for example, in EP1389637, EP1527176, US7452987, US7893245 or US8324370.

Lipid Nanoparticles (LNP) were prepared at a total lipid to siRNA weight ratio of 20:1.

### c) LNP delivery to different cell types

### i) In vivo evaluation of LNP delivery of siRNA to liver

### Study 1 (animal study code A0953)

The aim of this study was to evaluate liver cell targeting mediated by Lipid Nanoparticle Formulations in accordance with the invention. In this sense, 2 different formulations are tested, Lipid Nanoparticle Formulation 1 (LNF1) and Lipid Nanoparticle Formulation 2 (LNF2), as described above. siRNA were designed for the following gene targets: TIE-2 (endothelial cell target) (Tie2 is an endothelium-specific receptor tyrosine kinase), Factor 7 (hepatocyte target) (coagulation factor *VII* (F7), expressed in hepatocytes and secreted to blood) and GFAP (stellate cell target) (The glial fibrillary acidic protein (GFAP), is expressed in the liver in a subpopulation of quiescent stellate cells). These examples represent potential therapeutic targets: Tie2 may be a target for antiangiogenic therapy, GAP in may be a target for Liver Fibrosis in Chronic Hepatitis C (hepatic stellate cells activation pathway) and F7 may be a target for anticoagulant therapy.

### Sequences for study:

sense (b) 5' -- 3'
antisense (a) 5' -- 3'
(bold **2'OMe mod**; *italic 2' fluoro mod)*
TIE-2 (TIE2-hm8)
   5'- c**c**a **u**ca u**u**u **g**cc c**a**g **a**u**a** u-3'
   5'- **a**u**a** u**c**u **g**g**g** c**a**a **a**ug a**u**g **g**-3'
GFAP (GFAP-m-3)
   5'- u**g**g **u**a**u** c**g**g **u**c**u** a**a**g **u**u**u** g-3'
   5'- **c**a**a** a**c**u **u**ag a**c**c **g**a**u** a**c**c **a**-3'
Factor 7 (Factor7-m-fluoro)
   5'- g**g***a* **u***c***a** *u***c***u* **c**a**a** *g***u***c* **u***u***a** *c-3'*
   5'- **g***u***a** *a***g***a* **c***u***u** *g***a***g* **a***u***g** *a**u**c* **c**-3'

**Table 1: List of formulations**

| Payload | **# LNP** | Mean size (nm) | PDI | Zeta (charge, mV) | EE% |
|---|---|---|---|---|---|
| TIE2-hm8 | GN2801 (LNF1) | 108 | 0.03 | -8 | 94.8 |
| GFAP-m-3 | GN2802 (LNF1) | 98 | 0.03 | -9 | 95.5 |
| Factor7-m-fluoro | GN2803 (LNF1) | 158 | 0.13 | -6 | 97.7 |
| TIE2-hm8 | GN2804 (LNF2) | 133 | 0.04 | -6 | 96.0 |
| GFAP-m-3 | GN2805 (LNF2) | 153 | 0.06 | -5 | 94.0 |
| Factor7-m-fluoro | GN2806 (LNF2) | 164 | 0.06 | -4 | 96.7 |

In this table, the suffix indicates if the siRNA sequence targets mouse (m) rat (r) Cynomolgus (c) and/or human (h) transcripts. Fluoro modification is known to increase enzymatic stability in siRNA sequences

### Mice experiment design

A total of 36 mice (male C57BL/6JO1aHsd, 8 weeks) were allocated to groups (n=4). The mice were treated with a single injection dose of 0.5 mg/kg of siRNA formulated in LNP1 or LNF2 LNP (Table 2). In the case of F7, a lower 0.25 mg/kg was also tested (Table 2). Body weights were determined prior to dosing and at sacrifice to indicate the animals' health status. The mice were observed for any signs of ill-health and local intolerability at the injection site following treatment. After 48 h, animals were sacrificed. The organs were collected including liver, lung, spleen and heart and stored at -80 °C. The mRNA levels of the target gene levels were measured by TaqMan. Approximately 20 mg of tissue was homogenized in a Mixer Mill MM 301 (Retsch GmbH, Haan, Germany) using tungsten carbide beads (Qiagen, Hilden, Germany). Total RNA was isolated from the lysate with the Invisorb Spin Tissue RNA Mini Kit (Invitek, Berlin, Germany). Depending on the tissue, 25-100 ng total RNA was used for quantitative TaqMan RT-PCR. Data were normalized to an endogenous control (actin mRNA). The TaqMan RT-PCR reactions were carried out with an ABI PRISM 7700 Sequence Detector (Software: Sequence Detection System v1.6.3 (ABI Life Technologies)) or StepOnePlus Real Time PCR Sytem (ABI) using a standard protocol for RT-PCR as described previously (Santel, A, Aleku, M, Keil, O, Endruschat, J, Esche, V, Fisch, G et al. (2006) Gene Ther 13: 1222-1234) with primers and probes at a concentration of 300 and 100 nmol/l respectively.TaqMan data were calculated by using the comparative Ct method. Data were normalized to an endogenous control (actin mRNA).

**Table 2: Experimental groups**

| Group | Payload | | Delivery system | | Treatment route | Animals |
|---|---|---|---|---|---|---|
| | Designation | mg/kg | Designation | | | |
| A | HEPES Sucrose | - | - | | i.v. | 4 |
| B | TIE2-hm8 | 0.5 | LNF1 | GN2801 | i.v. | 4 |
| C | GFAP-m-3 | 0.5 | LNF1 | GN2802 | i.v. | 4 |
| D | Factor7-m-fluoro | 0.5 | LNF1 | GN2803 | i.v. | 4 |
| E | Factor7-m-fluoro | 0.25 | LNF1 | GN2803 | i.v. | 4 |
| F | Tie-2 | 0.5 | LNF2 | GN2804 | i.v. | 4 |
| G | GFAP-m-3 | 0.5 | LNF2 | GN2805 | i.v. | 4 |
| H | Factor7-m-fluoro | 0.5 | LNF2 | GN2806 | i.v. | 4 |
| I | Factor7-m-fluoro | 0.25 | LNF2 | GN2806 | i.v. | 4 |

### Results

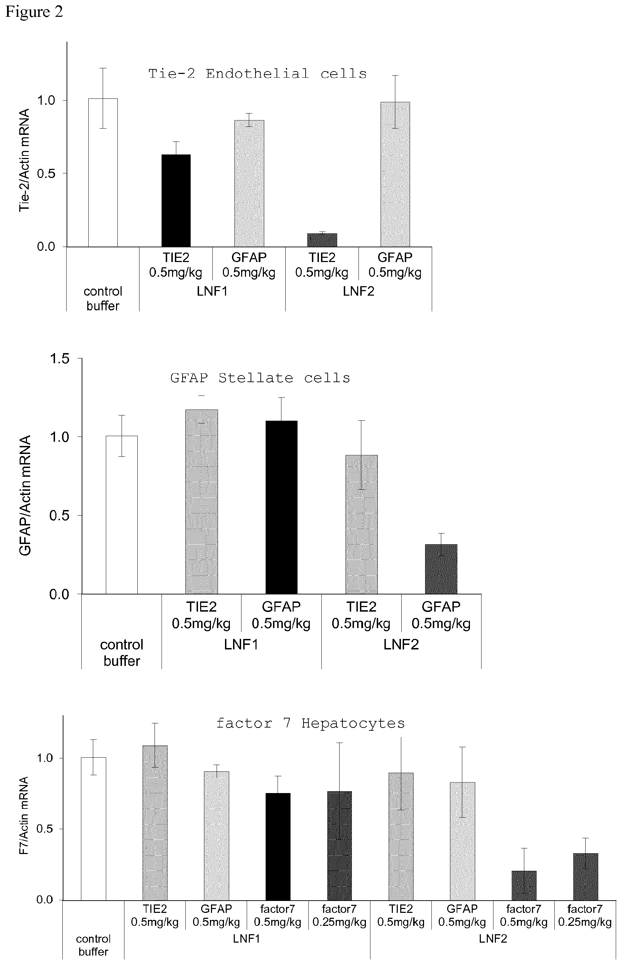

The siRNA delivery system LNF2 targets hepatocytes, endothelial cells and stellate cells in the liver. TaqMan analysis of liver lysates is shown in Figure 2. The siRNA LNF2 targets hepatocytes, endothelial cells and stellate cells in the liver as demonstrated by Tie-2, GFAP and F7 knockdown (KD) (Figure2). Percentage of KD 91%, 69% and 80%, respectively (vs buffer control). No silencing activity for LNF1 LNP. In the case of F7 in LNF2, an increase in the dose from 0.25mg/kg to 0.5mg//kg showed dose response increase KD from 67% to 80%.

### ii) In vivo evaluation of LNP delivery of siRNA to other organs.

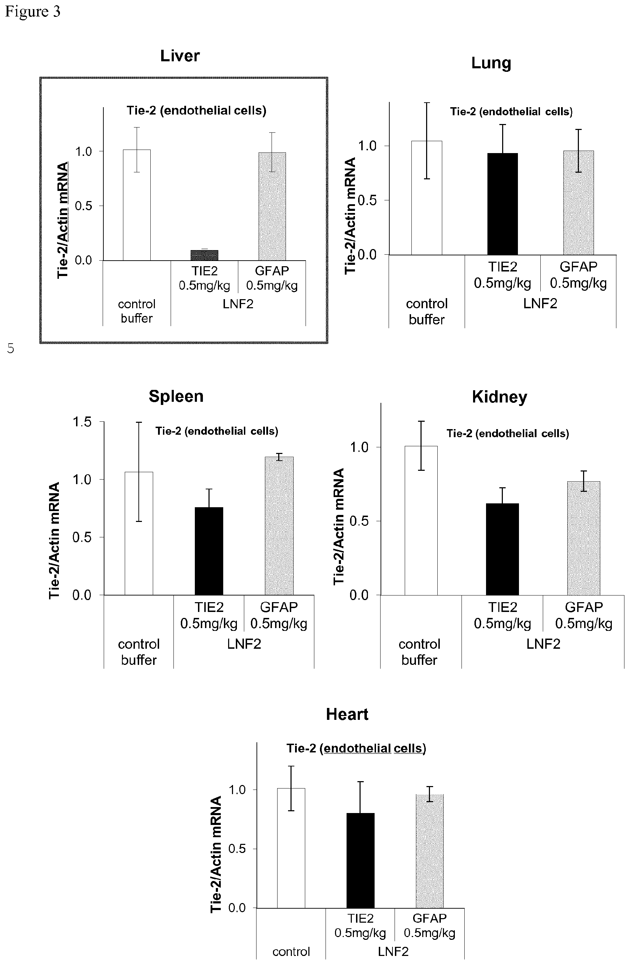

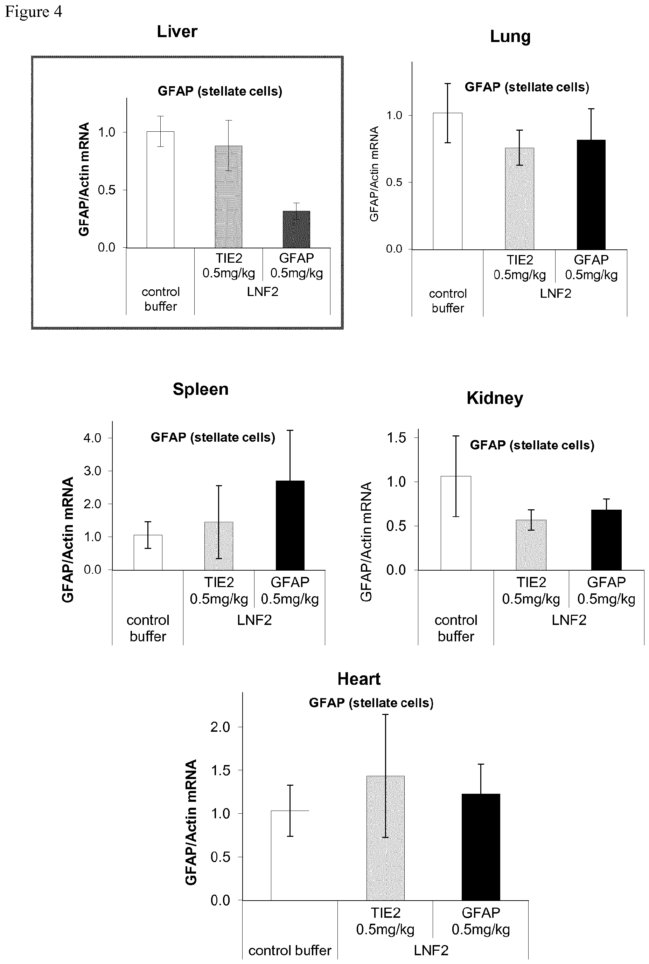

The siRNA delivery system Lipid Nanoparticle Formulation 2 (LNF2) targets cells in the liver. The analysis of Tie-2 expression and GFAP in organs other than liver (lung, spleen, kidney, heart) revealed no evidence of extra-hepatic knock-down mediated by LNP. TaqMan analysis of organ lysates are presented in Figures 3 and 4. Data normalized to Actin. Data was repeated with Lipid Nanoparticle Formulation 1 (LNF1) and no activity was observed in any organ, data not presented.

### Study 2 (animal study code A0957)

The aim of this study was to evaluate additional liver cell targeting mediated by siRNA Lipid Nanoparticle Formulation 2 (LNF2). siRNA were designed for the following gene targets: ApoB (apolipoprotein B-100) (hepatocyte target for atherosclerosis and cardiovascular diseases), Col1A1 (Collagen1) (stellate cells; the principal collagen in fibrotic liver and is expressed by in the liver), ITGB6 (Integrin Subunit Beta 6) (cholangiocytes; is uniquely upregulated in activated cholangiocytes and has an important role in the progression of biliary fibrosis), PDGF-R beta (platelet-derived growth factor receptor beta) (pericytes; marker), PDGF-R-alpha (platelet-derived growth factor receptor α (PDGFRα)) (various cell types broadly expressed, i.e. SM cells, fibroblasts, ECs; involved liver development, regeneration, and pathology-including fibrosis, cirrhosis, and liver cancer), CD68 (Cluster of Differentiation 68) (macrophage target; expressed in macrophages, monocytes, Kupffer cells), IL4R2 (macrophage target) and CD45 (Cluster of Differentiation 45) (leucocytes). siRNA modification according to published patterns as described, for example, in EP1389637B1, EP1527176B1, US7452987B2, US7893245B2 and US8324370B2, in some cases including fluoro modification.

**Table 3: List of Oligos**

| | |
|---|---|
| ApoB (ApoB hcm2 fluoro) | 5'-**u***u***a** *g***u***a* **a***a***c** *c***u***u* **u***u***g** *a***g***a* **c**-3' |
| | 5'-*g***u***c* **u***c***a** *a***a***a* **g***g*u *u***u***a* **c***u***a** *a*-3' |
| Col1A1 (CollA1-hm2) | 5'-u**g**g **u**g**a** a**c**c **u**gg c**a**a **a**c**a** a-3' |
| | 5'-**u**u**g** u**u**u **g**c**c** a**g**g **u**u**c** a**c**c **a**-3' |
| Col1 A1-hm2-fluoro | *5'-u***g***g* **u***g***a** *a***c***c* **u***g***g** *c***a***a* **a***c***a** a-3' |
| | 5'-**u***u***g** *u***u***u* **g***c***c** *a***g***g* **u***u***c** *a***c***c* **a**-3' |
| ITGB6 (ITGB6-mr12) | 5'-c**g**g **c**c**a** a**c**u **c**a**u** u**g**a **u**a**a** a -3' |
| | 5'-**u**uu a**u**c **a**a**u** g**a**g **u**u**g** g**c**c **g** -3' |
| PDGF-R alpha (PDGF-R-alpha-m1fluoro) | 5'-*c***u***g* **c***a***a** *g***c***a* **u***a***u** *u***a***a* **g***a***a** *a* -3' |
| | 5'- **u***u***u** *c***u***u* **a***a***u** a***u***g **c***u***u** *g***c***a* **g** -3' |
| PDGF-R beta (PDGF-R beta-m4-fluoro) | 5'- *g***a***g* **c***a***a** *g***g***a* **u***g***a** *a***u***c* **u***a***u** *a* -3' |
| | 5'- **u***a***u** *a***g***a* **u***u***c** *a***u***c* **c***u***u** *g***c***u* **c** -3' |
| CD68 (CD68-fluoro) | 5'- *a**c**u* **a***c***a** *u***g***g* **c***g***g** *u***g***g* **a***a***u** *a* -3' |
| | 5'- **u**a**u** *u***c***c* **a***c***c** *g**c**c **a**u***g** *u***a***g* **u** -3' |
| ILR2 (IL4R2-m-5-fluoro) | 5'- c**a**u **g***g**a** g***c***u* **u***u***a** *u***g***u* **c***a***a** *u* -3' |
| | 5'- **a***u***u** *g***a***c* **a***u***a** *a***a***g* **c***u***c** *c***a***u* **g**-3' |
| CD45 (CD45-hcmr-11-fluoro) | 5'- *u***g***a* **u***u***a** *u***u***g* **u***g***a** *c***a***u* **c***a***a** *u*-3' |
| | 5'- **a***u***u** *g***a***u* **g***u***c** *a***c***a* **a***u***a** *a***u***c* **a**-3' |

| | |
|---|---|
| sense (b) 5' -- 3' antisense (a) 5' -- 3' (bold **2'OMe mod;** *italic 2' fluoro mod)* | |

**Table 4: List of formulations**

| **Payload** | **# LNP** | Mean size (nm) | PDI | Zeta (charge, mV) | EE% |
|---|---|---|---|---|---|
| ApoB hcm2 fluoro | GN3101 (LNF2) | 147 | 0.03 | -0.57 | 98.6 |
| Col1A1-hm2 | GN3102 (LNF2) | 138.5 | 0.03 | -5.36 | 97.4 |
| Col1A1-hm2-fluoro | GN3103 (LNF2) | 138 | 0.08 | -3.41 | 97.7 |
| ITGB6-mr12 | GN3104 (LNF2) | 127.5 | 0.05 | -4.82 | 97.3 |
| PDGF-R-alpha-m1 | GN3105 (LNF2) | 142 | 0.02 | -5.17 | 97.3 |
| PDGF-R beta-m4-Fluoro | GN3106 (LNF2) | 155 | 0.05 | -6.67 | 96.8 |
| CD68-fluoro | GN3107 (LNF2) | 137 | 0.04 | -6.1 | 98.2 |
| IL4R2-m-5-Fluoro | GN3108 (LNF2) | 154 | 0.04 | -9.7 | 95.3 |
| CD45-hcmr-11 | GN3109 (LNF2) | 142.5 | 0.04 | -7.3 | 95.6 |

### Mice experiment design

A total of 40 mice (male C57BL/6JO1aHsd, 8 weeks) were allocated to groups (n=4). The mice were treated with a single injection dose of 0.5mg/kg of siRNA formulated in Lipid Nanoparticle Formulation 2 (LNF2). siRNA formulated in LNPs was administered via bolus tail-vein injection. Body weights were determined prior to dosing and at sacrifice to indicate the animals' health status. The mice were observed for any signs of ill-health and local intolerability at the injection site following treatment. After 48h, animals were sacrificed. The liver tissue was collected and stored at -80°C. The mRNA levels of the target gene levels were measured by TaqMan as described elsewhere. Data were normalized to an endogenous control (actin mRNA).

**Table 5: Experimental groups**

| **Group** | **Payload** | | **Delivery system** | **Treatment route** | **Animals** |
|---|---|---|---|---|---|
| | **Designation** | **mg/kg** | **Designation** | | |
| | control | | - | | |
| **A** | (HEPES/Sucrose) | - | | i.v. | 4 |
| **B** | ApoB hcm2-fl | 0.5 | LNF2 (GN3101) | i.v. | 4 |
| **C** | Col1A1-hm2 | 0.5 | LNF2 (GN3102) | i.v. | 4 |
| **D** | Col1A1-hm2-fl | 0.5 | LNF2 (GN3103) | i.v. | 4 |
| **E** | ITGB6 | 0.5 | LNF2 (GN3104) | i.v. | 4 |
| **F** | PDGF-R-alpha-ml | 0.5 | LNF2 (GN3105) | i.v. | 4 |
| **G** | PDGF-R beta-m4-fl | 0.5 | LNF2 (GN3106) | i.v. | 4 |
| **H** | CD68-fl | 0.5 | LNF2 (GN3107) | i.v. | 4 |
| **I** | IL4Ra-fl | 0.5 | LNF2 (GN3108) | i.v. | 4 |
| **J** | CD45-hcmr-11 | 0.5 | LNF2 (GN3109) | i.v. | 4 |

### Results

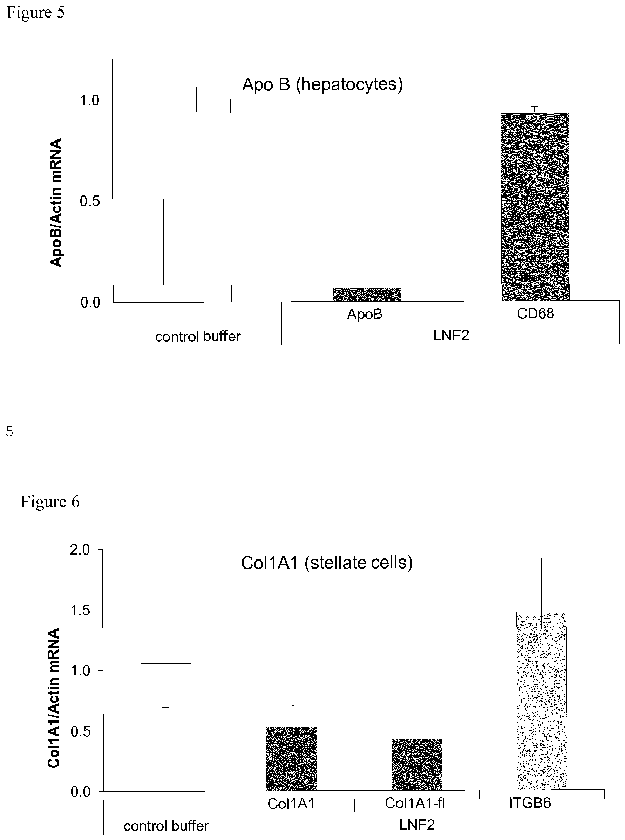

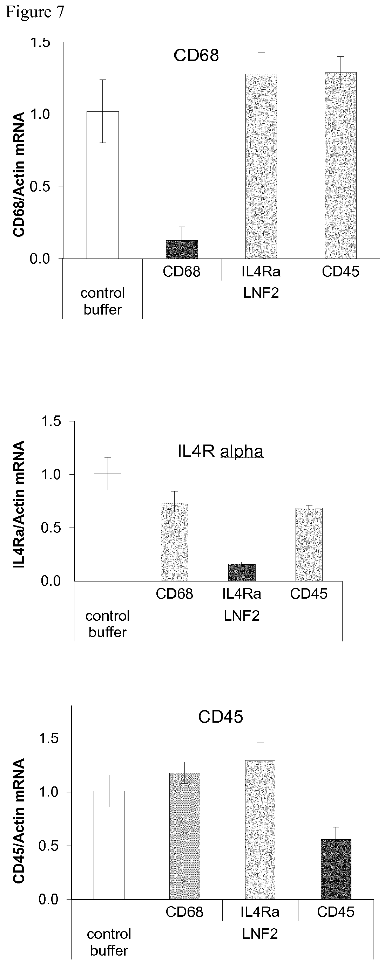

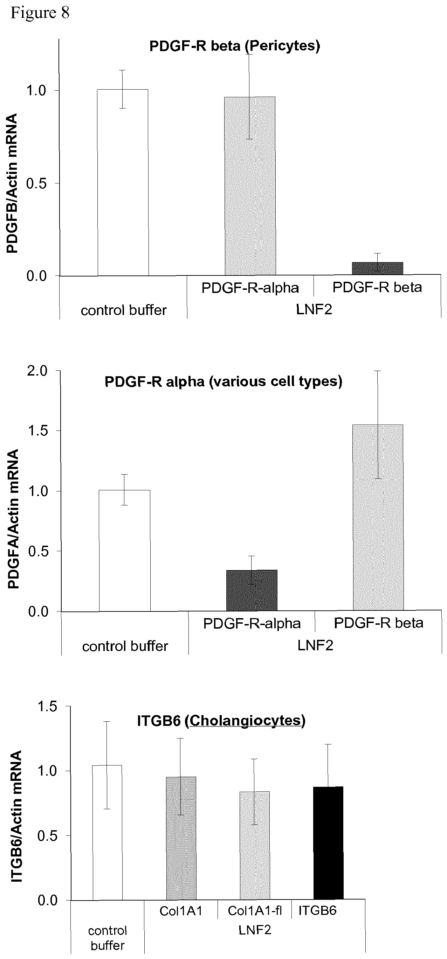

Lipid Nanoparticle Formulation 2 (LNF2) siRNA delivery system mediates inhibition of targets expressed in different cell types of the liver. TaqMan analysis of liver lysates is shown in Figures 5, 6, 7 and 8. Data represent group mean (n = 4) ± SD. The inhibition of targets expressed in different cell types of the liver was shown, including Hepatocytes (ApoB), Stellate Cells/Pericytes (Col1A1, PDGF-R beta), Macrophages (CD68, IL4R alpha), Neutrophils (CD45). Apparently, cholangiocytes (ITGB-6) are not targeted by this delivery system. Direct comparison of fluorinated and non-fluorinated siRNA Col1a1 showed no difference in silencing potency (Figure 6).

### Study 3 (animal study code A0995)

The aim of this study_was to evaluate additional liver cell targeting mediated by siRNA Lipid Nanoparticle Formulation 2 (LNF2). siRNA was designed for the following gene targets: TTR (Transthyretin; hepatocyte target) and Glul (Glutamine synthase;pericentral hepatocytes acting as stem cells in healthy liver). siRNA modification according to published patterns as described, for example, in EP1389637B1, EP1527176B1, US7452987B2, US7893245B2 and US8324370B2, in some cases including fluoro modification.

**Table 6: List of oligos**

| | |
|---|---|
| TTR (TTR-CF2) | 5'- *a****a****c* **a***g***u** *g***u***u* **c***u***u** *g***c***u* **c***u***a** *u***a***a* -3' |
| | 5'- **u****u****a** *u***a***g* **a***g***c** *a***a***g* **a***a***c** *a***c***u* **g****u****u*** -3' |
| Glul (Glul-hcmr6) | 5'- c**c**u **c**c**a** a**c**a **u**c**a** a**c**g **a**c**u** u -3' |
| | 5'- **a**a**g** u**c**g **u**u**g** a**u**g **u**ug g**a**g **g** -3' |

| | |
|---|---|
| sense (b) 5' -- 3' antisense (a) 5' -- 3' (bold **2'OMe mod;** *italic 2' fluoro mod,* Asterisk indicates phosphothioate (P=S) modification) | |

**Table 7: List of formulations**

| Payload | **# LNP** | Mean size (nm) | PDI | Zeta (charge, mV) | EE% |
|---|---|---|---|---|---|
| TTR-CF02 | GN5301 LNF2 | 121 | 0.05 | -3.2 | 95.8 |
| Glul-hcmr-6 | GN5302 LNF2 | 160 | 0.05 | -2.2 | 93.2 |

### Mice experiment design

A total of 24 mice (male C57BL/6JO1aHsd, 8 weeks) were allocated to groups (n=6). The mice were treated with a single injection dose of 1 or 0.5mg/kg of siRNA formulated in Lipid Nanoparticle Formulation 2 LNP (LNF2). siRNA formulated in LNPs were administered via bolus tail-vein injection. Body weights were determined prior to dosing and at sacrifice to indicate the animals' health status. The mice were observed for any signs of ill-health and local intolerability at the injection site following treatment. After 48h, animals were sacrificed. The liver tissue was collected and stored at -80°C. The mRNA levels of the target gene levels were measured by TaqMan analysis as described elsewhere. Data were normalized to an endogenous control (actin mRNA).

**Table 8: Experimental groups**

| **Group** | **Payload** | | **Delivery system** | **Treatment route** | **Animals** |
|---|---|---|---|---|---|
| | **Designation** | **mg/kg** | **Designation** | | |
| **A** | TTR-CF2 | 0.5 | "LNF02" (GN5301) | i.v. | 6 |
| **B** | Glul-hcmr6 | 0.5 | "LNF02" (GN5302) | i.v. | 6 |
| **C** | TTR-CF2 | 1.0 | "LNF02" (GN5301) | i.v. | 6 |
| **D** | Glul-hcmr6 | 1.0 | "LNF02" (GN5302) | i.v. | 6 |

### Results

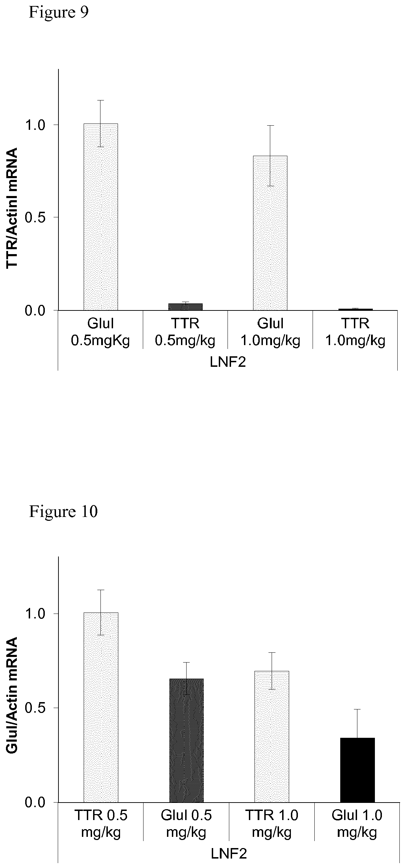

Lipid Nanoparticle Formulation 2 (LNF2) siRNA delivery system mediates inhibition of targets expressed in different cell types of the liver. TaqMan analysis of liver lysates is shown in Figures 9 and 10. Data represent group mean (n = 6) ± SD. The inhibition of targets expressed in different cell types of the liver was shown, including Hepatocytes (TTR) and stem cells (Glul).

### d) Immune response to LNF 2 delivery

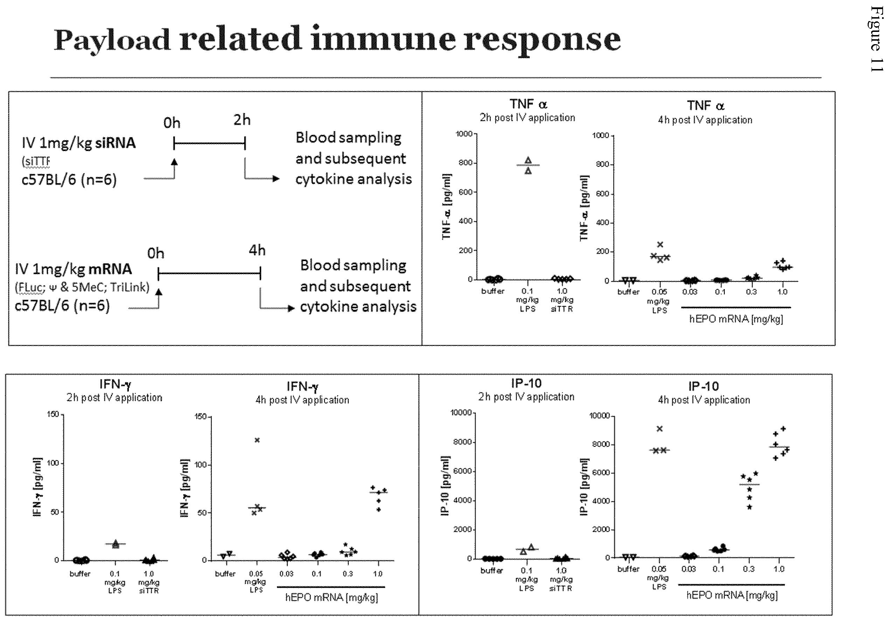

For cytokine analysis c57BL/6 mice were either i.v. treated with Lipid Nanoparticle Formulation (2) delivering siRNA or mRNA or left untreated. Lipopolysaccharide (LPS) treatment was included as a positive control. At an early time point (2h or 4h) following i.v. treatment blood was drawn under anaesthesia via retro-orbital puncture with capillaries and sampled into EDTA coated tubes (Sarsted). For generation of EDTA-plasma, samples were centrifuged at 4°C for 10min at 800xg, supernatants were transferred into Eppendorf tubes and stored at -80°C until analysis. Cytokines were quantified according to the manufacturer's instructions with a ProcartaPlex [Mouse Cytokine & Chemokine Panel 1A (36 plex)] from eBioscience in a MagPix (BioRad) instrument. In detail EDTA-plasma was analysed either undiluted or 10 times diluted in assay diluent in order to reach values within the range of the standard curve. Results for three cytokines are shown in Figure 11.

### e) CRISPR mRNA formulations

### i) Preparation of LNP systems

LNP were prepared by mixing appropriate volumes of lipid stock solutions in ethanol buffer with an aqueous phase containing appropriate amounts of guide RNA (sgRNA) and Cas9 mRNA (as described below) employing a microfluidic mixer as NanoAssemblr (Precision Nanosystems).

For the encapsulation of sgRNA/mCas9 payloads, the desired amount of such mixtures was dissolved in 25 mM sodium acetate, pH 4.0. Then, 3 volumes of the lipid in ethanol and 1 volume of the nucleic acids in water were combined in the microfluidic mixer (Nanoassembler, Precision Nanosystems, Canada) using a dual syringe pump to drive the solutions through the microfluidic cartridge at a combined flow rate of 18 ml/min reducing the ethanol content of the mixture to 25%. The lipid mixture was then dialyzed for 4 hours against 10mM HEPES 9% Sucrose (2 cycles) and followed by an overnight dialysis using dialysis membranes (molecular weight cutoff 3500 Da, Slide-A-Lyzer™ Dialysis Cassettes, Thermofisher). The mean diameter and zeta potential of the LNP after dialysis was as determined by dynamic light scattering and Laser Doppler Micro-electrophoresis, respectively using a Zetasizer Nano ZS (Malvern). Encapsulation efficiency (EE%) was calculated by determining unencapsulated sgRNA/mCas9 content by measuring the fluorescence upon the addition of RiboGreen (Molecular Probes, Eugene, OR) to the LNP (Fi) and comparing this value to the total content that is obtained upon lysis of the LNP by 1% Triton X-100 (Ft): % encapsulation = (Ft - Fi)/Ft x 100.

The following sequences are referred to in the below examples:
Cas 9 mRNA (incl. UTRs) from Trilink (L-6125, Cas9 mRNA (5meC, Ψ)).

The TTR guide RNA (sgTTR) used had the following sequence wherein modifications are as indicated.

U*C*A*CAGGAUCACUCACCGCGGUUUUAGAGCUAGAAAUAGCAAGUUAA
AAUAAGGCUAGUCCGUUAUCAACUUGAAAAAGUGGCACCGAGUCGG*U*G
*C

The PCSK9n guide RNA (sgPCSK9) used had the following sequence wherein modifications are as indicated:

A*C*A*GUCAGCGGCACCCUCAUGUUUUAGAGCUAGAAAUAGCAAGUUAA
AAUAAGGCUAGUCCGUUAUCAACUUGAAAAAGUGGCACCGAGUCGG*U*G
*C

The GFP-specific guide RNA sequence (20 nt target specific sequence for GFP: GGAGCGCACCATCTTCTTCA) and sequence of single chimeric guide RNA specific for *Streptococcus pyogenes* Cas9 has been described (Mali et al., Science. 2013 February 15; 339(6121): 823-826 and Jinek et al., Science. 2012 Aug 17;337(6096):816-21, respectively) and the methods are incorporated herein by reference.

In the above sequences, bases underlined are 2'-O-methyl modified; * represent phophorothioates, (wherein the * is positioned to the right of the nucleotide having the modification e.g. G*U indicates that the G is modified).

### ii) Formulation of LNP at several guideRNA:mRNACas9 ratios

Cationic lipid (Coatsome SS-33/4PE-15), DPhyPE, cholesterol and PEG-lipid were solubilized in alcoholic solution at a molar ratio of 59:30:10:1. Coatsome SS-33/4PE-15 was prepared at 30 mg/ml in t-ButOH, all other at 20 mg/ml, dissolved in ethanol and filter sterilized through 0.2 µm syringe filters. Lipid Nanoparticles (LNP) were prepared at a total lipid to mRNA weight ratio of 40:1. Briefly the sgRNA/mCas9 at ratios (1:3, 1:9, 1:19) (= 25%, 10% and 5% respectively of guide RNA per total payload content) were diluted in 50 mM Acetate buffer, pH4 and mixed with the lipid solution as described. Lipid Nanoparticle size, zeta potential and EE% were measured as previously described) (Table 9).

**Table 9: LNP at several guideRNA: mRNACas9 ratio**

| Payload | **# LNP** | Mean size (nm) | PDI | Zeta (charge, mV) | EE% |
|---|---|---|---|---|---|
| mRNA-mCas9 75% + 25% sgPCSK9 | GN2601 | 130 | 0.15 | -1.74 | 100 |
| mRNA-mCas9 90% + 10% sgPCSK9 | GN2602 | 130 | 0.08 | -2.91 | 100 |
| mRNA-mCas9 95% + 5% sgPCSK9 | GN2603 | 120 | 0.08 | -1.26 | 99 |
| mRNA-mCas9 75% + 25% sgGFP | GN2604 | 158 | 0.08 | -5.67 | 97 |
| mRNA-mCas9 90% + 10% sgGFP | GN2605 | 120 | 0.126 | -0.84 | 100 |
| mRNA-mCas9 95% + 5% sgGFP | GN2606 | 135 | 0.124 | -2.76 | 100 |

### iii) Formulation of LNP with mRNA, sgRNA or siRNA payloads

Cationic lipid (Coatsome SS-33/4PE-15), DPhyPE, cholesterol and PEG-lipid were solubilized in alcoholic solution at a molar ratio of 59:30:10:1. Coatsome SS-33/4PE-15 was prepared at 30 mg/ml in t-ButOH, all others at 20 mg/ml, dissolved in ethanol and filter sterilized through 0.2 µm syringe filters. Lipid Nanoparticles (LNP) were prepared at a total lipid to mRNA weight ratio of 20:1 to 45:1. Briefly the nucleic acids were diluted in 50 mM Acetate buffer, pH4 and mixed with lipid solution as described. Lipid Nanoparticle size, zeta potential and EE% were measured as previously described (Table 10).

**Table 10: LNP with mRNA, sgRNA or siRNA payloads**

| **Payload** | **#LNP** | m/n Lipid/Oligo ratio | Mean size (nm) | PDI | Zeta (charge, mV) | EE% Condensation |
|---|---|---|---|---|---|---|
| mRNA Cas9 100% | GN4301 | 45 | 89 | 0.33 | -8.4 | 96.6 |
| sgTTR 100% | GN4302 | 20 | 114 | 0.14 | -23 mV (indicates not complete condensation of gRNA) | 20 |
| | GN4303 | 45 | 200 | 0.04 | -6.64 mV | 85.3 |
| Apo B siRNA 100% | GN3101 | 20 | 147 | 0.03 | -0.57 | 98.6 |

| | | | | | | |
|---|---|---|---|---|---|---|
| Note Table 9 vs Table 10 - better EE% of co-encapsulated mCas9/sgRNA LNP than individually encapsulated LNP. | | | | | | |

### f) LNP delivery of CRISPR formulations to cells

### i) In vitro evaluation of LNP delivery of Cas9 mRNA and synthetic guide RNAs to liver for gene disruption of murine TTR

*In vitro* transfection of different guide RNA to mRNA ratios in Hepa 1-6 and primary hepatocytes. Hepa 1-6 cells and primary hepatocytes were transfected with different ratios of guide RNA (gRNA/sgRNA) and mRNA Cas9 (mCas9). A constant amount of Cas9 mRNA was mixed with increasing amounts of guide RNA (sgTTR). The sgGFP and mRNA mCherry (Trilink L-6113 mCherry mRNA (5meC, Ψ)) were used as controls. The cells were transfected with commercial reagent 'Lipofectamine® MessengerMAX™ Transfection Reagent' (ThermoFischer Scientific) according to manufacturer's instructions. The in vitro disruption of the TTR gene was analyzed by genomic TaqMan assay. Genomic DNA from cells was isolated from the lysate with the DNAeasy blood+tissue kit (Quiagen) 1.5 micrograms total DNA was used for quantitative TaqMan PCR. Data were normalized to an endogenous control (actin mRNA). The TaqMan PCR reactions were carried out with an ABI PRISM 7700 Sequence Detector (Software: Sequence Detection System v1.6.3 (ABI Life Technologies)) or StepOnePlus Real Time PCR Sytem (ABI) using a standard protocol for RT-PCR as described previously excluding the reverse transcription step (Santel, A, Aleku, M, Keil, O, Endruschat, J, Esche, V, Fisch, G et al. (2006) Gene Ther 13: 1222-1234) with primers and probes at a concentration of 300 and 100 nmol/l respectively. TaqMan data were calculated by using the comparative Ct method.

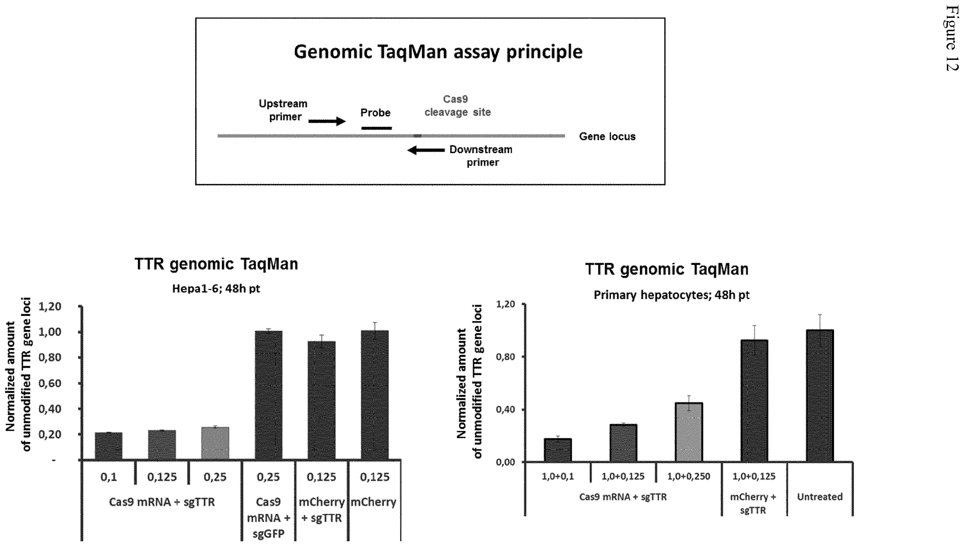

Figure 12 shows *in vitro* disruption of the TTR gene in Hepa 1-6 and primary Hepatocytes after transfection with different mCas9 + sgRNA ratios.

### ii) In vivo evaluation of LNP delivery of Cas9 mRNA and synthetic guide RNAs to liver for gene disruption of murine TTR.

Background: In vitro disruption of TTR gene in Hepa 1-6 and primary Hepatocytes was observed after transfection with different mCas9 + sgRNA ratios. The amount of mRNA-Cas9 is the limiting factor for maximum CRISPR mediated gene editing (Figure 12). In addition, an LNP delivery system, has previously shown high liver tropism for siRNA/mRNA delivery (i.e >90% mRNA expression in liver, TTR KD IC50≈ 0.06 mg/kg after single i.v siRNA-LNP). Finally, the co-loading of sgRNA and mCas9 gave 100-160 nm particles with better encapsulation efficiency than individual mRNA-Cas9 LNP and sgRNA LNP (Table 9 and Table 10).

The aim of this study was the evaluation of simultaneous delivery of sgRNA and mRNA-Cas9 in a single LNP system (co-encapsulating sgRNA and mCas9) for CRISPR-mediated gene disruption of TTR and PCSK9 genes in the liver.

**Table 11: List of formulations**

| **Payload mCas 9 : sgRNA (9:1 ration)** | #LPN | m/m Lipid/Oligo ratio | Mean size (nm) | PDI | Zeta (charge, mV) | EE% Condensation |
|---|---|---|---|---|---|---|
| mRNA-mCas9 (5mC & ψ) + gTTR-double-guide63 | AR054-01B | 45 | 119.6 | 0.037 | 2.66 | 100 |
| mRNA-mCas9 (5mC & ψ) + gTTR-double-guide28 | AR054-02B | 45 | 118.3 | 0.017 | 1.05 | 99.4 |
| mRNA-mCas9 (5mC & ψ) + mPCSK9-guide63 PS | AR054-03B | 45 | 135 | 0.038 | 1.13 | 99.2 |
| mRNA-mCas9 (5mC & ψ) + mPCSK9-guide28 | AR054-04B | 45 | 116.3 | 0.036 | 1.22 | --- |

Wherein guide28 and guide63 are different guide modification patterns, such as those described in PCT/GB2016/053312, incorporated herein by reference.

### Mice experiment design

A total of 25 mice (male C57BL/6JO1aHsd, 8 weeks) were allocated to five groups. The mice were treated once weekly for 4 weeks at doses of 1.1 mg/kg (week 1) and 0.5mg/kg (weeks 2, 3 and 4). Body weights were determined prior to dosing and at sacrifice to indicate the animals' health status. The mice were observed for any signs of ill-health and local intolerability at the injection site following treatment.

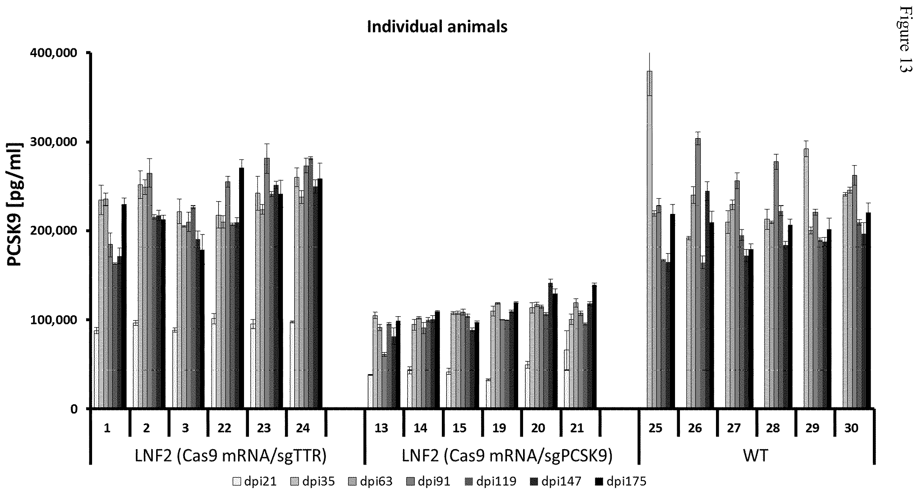

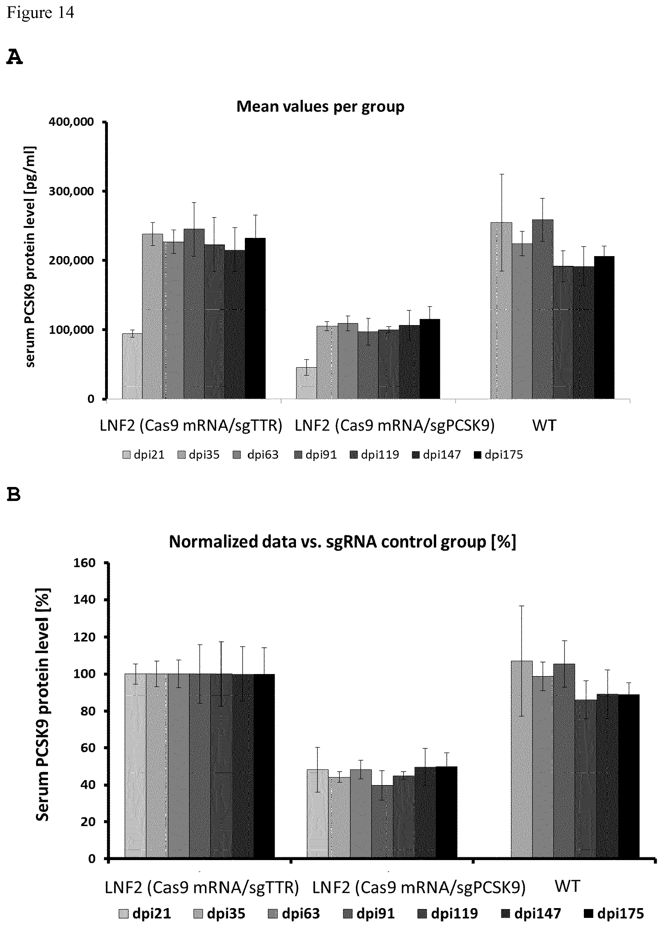

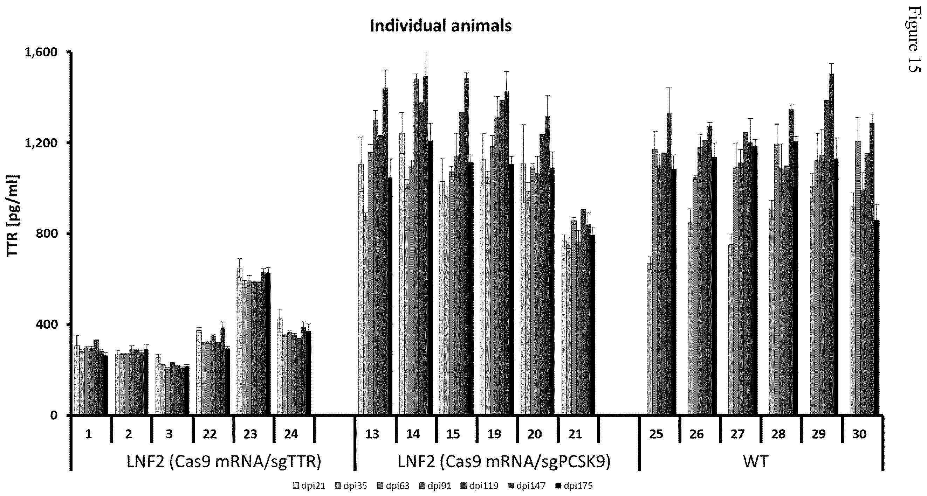

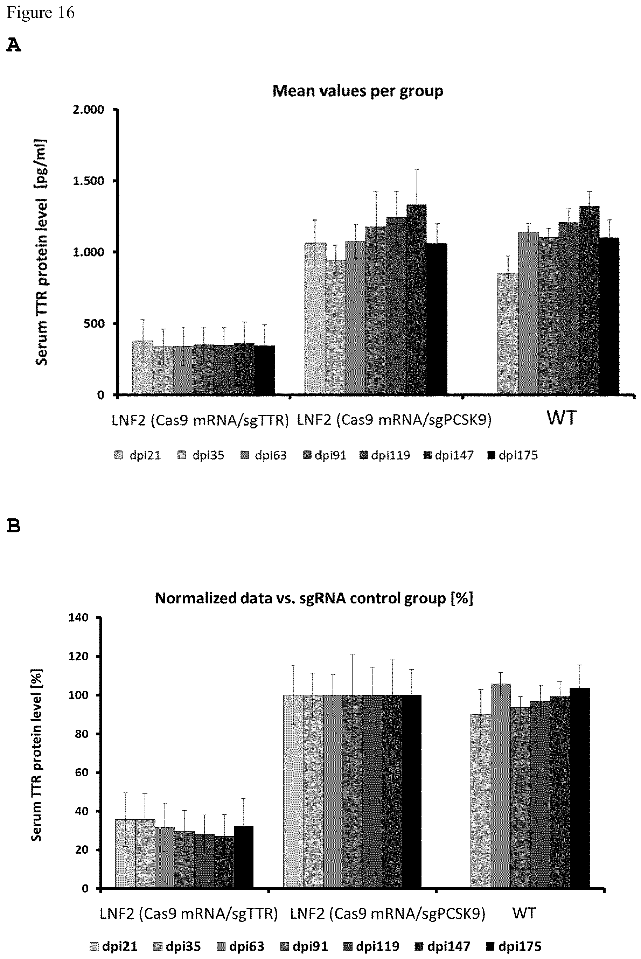

Blood samples were taken on days 21, 35, 63, 91, 119, 147 and 175 post first injection. Serum was obtained, aliquoted and stored at -80°C. TTR and PCSK9 levels were measured by Western Blot (d21) and ELISA kit (d21-175). Experimental results are presented in Figures 13, 14, 15 and 16.

## Claims

1. A formulation comprising:
i) a cationic lipid, or a pharmaceutically acceptable salt thereof;
ii) a steroid;
iii) a phosphatidylethanolamine phospholipid;
iv) a PEGylated lipid.

2. A formulation according to claim 1 wherein the content of the cationic lipid component is from about 55 mol% to about 65 mol% of the overall lipid content of the lipid formulation, preferably about 59 mol% of the overall lipid content of the lipid formulation.

3. A formulation as claimed in any preceding claim, wherein the steroid is cholesterol.

4. A formulation as claimed in any preceding claim, wherein the content of the steroid is from about 26 mol% to about 35 mol% of the overall lipid content of the lipid formulation, preferably about 30 mol% of the overall lipid content of the lipid formulation.

5. A formulation as claimed in any preceding claim, wherein the phosphatidylethanolamine phospholipid is selected from 1,2-diphytanoyl-*sn-*glycero-3-phosphoethanolamine (DPhyPE), 1,2-dioleoyl-*sn*-glycero-3-phosphoethanolamine (DOPE), 1,2-distearoyl-*sn*-glycero-3-phosphoethanolamine (DSPE), 1,2-Dilauroyl-sn-glycero-3-phosphoethanolamine (DLPE), 1,2-Dimyristoyl-sn-glycero-3-phosphoethanolamine (DMPE), 1,2-Dipalmitoyl-sn-glycero-3-phosphoethanolamine (DPPE), 1,2-Dilinoleoyl-sn-glycero-3-phosphoethanolamine (DLoPE), 1-Palmitoyl-2-oleoyl-sn-glycero-3-phosphoethanolamine (POPE), 1,2-Dierucoyl-sn-glycero-3-phosphoethanolamine (DEPE), 1,2-Disqualeoyl-sn-glycero-3-phosphoethanolamine (DSQPE) and 1-Stearoyl-2-linoleoyl-sn-glycero-3-phosphoethanolamine (SLPE).

6. A formulation as claimed in any preceding claim, wherein the content of the phospholipid is about 10 mol% of the overall lipid content of the formulation.

7. A formulation as claimed in any preceding claim, wherein the PEGylated lipid is selected from 1,2-dimyristoyl-*sn*-glycerol, methoxypolyethylene glycol (DMG-PEG) and N-palmitoyl-sphingosine-1-{succinyl[methoxy(polyethylene glycol) (C16-Ceramide-PEG).

8. A formulation as claimed in claim 7, wherein the content of PEGylated lipid is from about 1 mol% to about 5 mol%, or form about 1 mol% to about 3 mol%, in particular about 1 mol% of the overall lipid content of the formulation.

9. A formulation as claimed in any preceding claim, wherein the molar ratio of i): ii): iii): iv) is selected from 55:34:10:1; 56:33:10:1; 57:32:10:1; 58:31:10:1; 59:30:10:1; 60:29:10:1; 61:28:10:1; 62:27:10:1; 63:26:10:1; 64:25:10:1; and 65:24:10:1.

10. A pharmaceutical composition comprising a formulation as claimed in any one of claims 1 to 9 and a pharmaceutically active compound and preferably a pharmaceutically acceptable carrier.

11. The formulation or pharmaceutical composition as claimed in any preceding claim wherein said formulation or composition is a lipid nanoparticle.

12. The formulation or composition as claimed in claims 10 or 11 wherein the pharmaceutically active compound is a peptide, a protein, an oligonucleotide, a polynucleotide or a nucleic acid.

13. The formulation or composition as claimed in claim 12 wherein the nucleic acid is DNA, RNA, PNA, LNA, optionally wherein the nucleic acid is an mRNA or a functional nucleic acid such as RNAi, siRNA, siNA, saRNA, antisense nucleic acid, ribozymes, aptamers, spiegelmers, gRNA, optionally wherein the nucleic acid molecule is a modified nucleic acid molecule, preferably a modified siRNA, mRNA or gRNA.

14. The formulation or composition as claimed in any of claims 10 to 12 wherein the formulation comprises a gRNA in conjunction with an mRNA encoding a CRISPR endonuclease.

15. The formulation or composition as claimed any preceding claim wherein said formulation targets cells in the liver but not other organs (e.g. lung, kidney, heart), optionally wherein said formulation targets hepatocytes, stellate cells/pericytes, macrophages, neutrophils, preferably wherein said formulation targets hepatocytes.

16. A method for preparing a formulation or pharmaceutical composition as claimed in any preceding claim comprising:
a) preparing a lipid stock solution comprising:
i) a cationic lipid, or a pharmaceutically acceptable salt thereof;
ii) a steroid;
iii) a phosphatidylethanolamine phospholipid;
iv) a PEGylated lipid; and
b) mixing said lipid stock solution with a pharmaceutically active compound in a microfluidic mixer to obtain said formulation or pharmaceutical composition.

17. A formulation or pharmaceutical composition as claimed in any one of claims 1 to 15 for use as a medicament.

18. A formulation or pharmaceutical composition as claimed in any one of claims 1 to 15 for use in the treatment of liver disease or a liver-related disorder.

19. A method of delivery of nucleic acids to cells in the liver using a formulation or composition as defined in any one of claims 1 to 15.
